# EUROPEAN PATENT SPECIFICATION

(11) **EP 1 390 535 B1**
(45) Date of publication and mention of the grant of the patent: **30.06.2010**
(21) Application number: 02725828.4
(22) Date of filing: 26.04.2002
(51) Int. Cl.: C12Q 1/68, C12P 21/06, C07K 16/00, G01N 33/68

(54) **COMBINATORIAL LIBRARIES OF MONOMER DOMAINS**
KOMBINATORISCHE BIBLIOTHEKEN VON MONOMERDOMÄNEN
BANQUES COMBINATOIRES DE DOMAINES MONOMERES

(30) Priority: 26.04.2001 US 286823 P; 19.11.2001 US 337209 P; 26.11.2001 US 333359 P; 18.04.2002 US 374107 P
(43) Date of publication of application: 25.02.2004
(73) Proprietor: Amgen Mountain View Inc., Wilmington DE 19801 (US)
(72) Inventor: KOLKMAN, Joost, A., 9830 Sint-Martens-Latem (BE); STEMMER, Willem, P.C., Los Gatos, CA 95030 (US)
(74) Representative: Mercer, Christopher Paul
(86) International application number: PCT/US2002/013257
(87) International publication number: WO 2002/088171

(56) References cited:
- WO-A-00/55207
- WO-A-94/04691
- WO-A-96/37621
- WO-A-99/06587
- WO-A-99/10506
- WO-A-99/20749
- US-A- 5 427 908
- US-B1- 6 329 507
- LI MIN: "Applications of display technology in protein analysis" NATURE BIOTECHNOLOGY, vol. 18, no. 12, December 2000 (2000-12), pages 1251-1256, XP002162318 ISSN: 1087-0156
- WHALEN R G ET AL: "DNA SHUFFLING AND VACCINES" CURRENT OPINION IN MOLECULAR THERAPEUTICS, CURRENT DRUGS, LONDON, vol. 3, no. 1, 2001, pages 31-36, XP000995221 ISSN: 1464-8431

## Description

### BACKGROUND OF THE INVENTION

Analysis of protein sequences and three-dimensional structures have revealed that many proteins are composed of a number of discrete monomer domains. The majority of discrete monomer domain proteins is extracellular or constitutes the extracellular parts of membrane-bound proteins.

An important characteristic of a discrete monomer domain is its ability to fold independently or with some limited assistance. Limited assistance can include assistance of a chaperonin(s) (e.g., a receptor-associated protein (RAP)).The presence of a metal ion(s) also offers limited assistance. The ability to fold independently prevents misfolding of the domain when it is inserted into a new protein environment. This characteristic has allowed discrete monomer domains to be evolutionarily mobile. As a result, discrete domains have spread during evolution and now occur in otherwise unrelated proteins. Some domains, including the fibronectin type III domains and the immunoglobin-like domain, occur in numerous proteins, while other domains are only found in a limited number of proteins.

Proteins that contain these domains are involved in a variety of processes, such as cellular transporters, cholesterol movement, signal transduction and signaling functions which are involved in development and neurotransmission. *See* Herz, Lipoprotein receptors: beacons to neurons?, (2001) Trends in Neurosciences 24(4):193-195; Goldstein and Brown, The Cholesterol Quartet, (2001) Science 292: 1310-1312. The function of a discrete monomer domain is often specific but it also contributes to the overall activity of the protein or polypeptide. For example, the LDL-receptor class A domain (also referred to as a class A module, a complement type repeat or an A-domain) is involved in ligand binding while the gamma-carboxyglumatic acid (Gla) domain which is found in the vitamin-K-dependent blood coagulation proteins is involved in high-affinity binding to phospholipid membranes. Other discrete monomer domains include, e.g., the epidermal growth factor (EGF)-like domain in tissue-type plasminogen activator which mediates binding to liver cells and thereby regulates the clearance of this fibrinolytic enzyme from the circulation and the cytoplasmic tail of the LDL-receptor which is involved in receptor-mediated endocytosis.

Individual proteins can possess one or more discrete monomer domains. These proteins are often called mosaic proteins. For example, members of the LDL-receptor family contain four major structural domains: the cysteine rich A-domain repeats, epidermal growth factor precursor-like repeats, a transmembrane domain and a cytoplasmic domain. The LDL-receptor family includes members that: 1) are cell-surface receptors; 2) recognize extracellular ligands; and 3) internalize them for degradation by lysosomes. See Hussain et al., The Mammalian Low-Density Lipoprotein Receptor Family, (1999) Annu. Rev. Nutr. 19:141-72. For example, some members include very-low-density lipoprotein receptors (VLDL-R), apolipoprotein E receptor 2, LDLR-related protein (LRP) and megalin. Family members have the following characteristics: 1) cell-surface expression; 2) extracellular ligand binding consisting of A-domain repeats; 3) requirement of calcium for ligand binding; 4) recognition of receptor-associated protein and apolipoprotein (apo) E; 5) epidermal growth factor (EGF) precursor homology domain containing YWTD repeats; 6) single membrane-spanning region; and 7) receptor-mediated endocytosis of various ligands.. *See* Hussain, *supra.* Yet, the members bind several structurally dissimilar ligands.

It is advantageous to develop methods for generating and optimizing the desired properties of these discrete monomer domains. However, the discrete monomer domains, while often being structurally conserved, are not conserved at the nucleotide or amino acid level, except for certain amino acids, e.g., the cysteine residues in the A-domain. Thus, existing nucleotide recombination methods fall short in generating and optimizing the desired properties of these discrete monomer domains.

The present invention addresses these and other problems.

### BRIEF SUMMARY OF THE INVENTION

The present invention provides a method for identifying a multimer that binds to a target molecule, the method comprising,
(a) providing a library of polypeptides, the polypeptides comprising different monomer domains, wherein each monomer domain has 30-100 amino acids, comprises at least two disulfide bonds, and is an LDL-receptor class A domain;
(b) screening the library of polypeptides for affinity to a target molecule;
(c) identifying at least a first polypeptide comprising a first monomer domain that specifically binds to a target molecule;
(d) linking the first monomer domain to a plurality of different monomer domains to form a library of different multimers, the multimers comprising the first monomer domain and one of the plurality of different monomer domains;
(e) screening the library of multimers for the ability to bind to the target molecule; and
(f) identifying a multimer that specifically binds to the target molecule, wherein the multimer comprises the first monomer domain and a second monomer domain.

In some embodiments, at least three monomer domains bind to the same target molecule.

Further described herein are methods comprising a further step of mutating at least one monomer domain, thereby providing a library comprising mutated monomer domains. The mutating step may comprise recombining a plurality of polynucleotide fragments of at least one polynucleotide encoding a monomer domain. The mutating step may comprise directed evolution. The mutating step may comprise site-directed mutagenesis.

Further described herein are methods that comprise: screening the library of monomer domains for affinity to a second target molecule; identifying a monomer domain that binds to a second target molecule; linking at least one monomer domain with affinity for the first target molecule with at least one monomer domain with affinity for the second target molecule, thereby forming a library of multimers; screening the library of multimers for the ability to bind to the first and second target molecule; and identifying a multimer that binds to the first and second target molecule, thereby identifying a multimer that specifically binds a first and a second target molecule.

Certain methods described herein further comprise: providing a second library of monomer domains; screening the second library of monomer domains for affinity to at least a second target molecule; identifying a second monomer domain that binds to the second target molecule; linking the identified monomer domains that bind to the first target molecule or the second target molecule, thereby forming a library of multimers; screening the library of multimers for the ability to bind to the first and second target molecule; and identifying a multimer that binds to the first and second target molecules.

The target molecule may be selected from the group consisting of a viral antigen, a bacterial antigen, a fungal antigen, an enzyme, a cell surface protein, an enzyme inhibitor, a reporter molecule, and a receptor. The viral antigen may be a polypeptide required for viral replication. The first and at least second target molecules may be different components of the same viral replication system. The selected multimer may bind to at least two serotypes of the same virus.

In some embodiments, the library of multimers is expressed as a phage display, ribosome display or cell surface display.

In some embodiments, the monomer domains are linked by a polypeptide linker. As described herein, the polypeptide linker may be a linker naturally-associated with the monomer domain. In some embodiments, the polypeptide linker is a variant of a linker naturally-associated with the monomer domain. The linking step may comprise linking the monomer domains with a variety of linkers of different lengths and composition.

The present invention also provides polypeptides comprising the multimers selected as described above.

The present invention also provides polynucleotides encoding the multimers selected as described above.

The present invention also provides libraries of multimers formed as described above.

The present invention also provides methods for identifying a multimer that binds to at least one target molecule, comprising the steps of: providing a library of multimers, wherein each multimer comprises at least two monomer domains and wherein each monomer domain comprises at least two disulfide bonds, has 30 - 100 amino acids, is a receptor LDL class A domain and the monomer domains are separated by a heterologous linker; and screening the library of multimers for target molecule-binding multimers. In some embodiments, the methods further comprise identifying target molecule-binding multimers having an avidity for the target molecule that is greater than the avidity of a single monomer domain for the target molecule. In some embodiments, one or more of the multimers comprises a monomer domain that specifically binds to a second target molecule.

The present invention also provides libraries of multimers. In some embodiments, each multimer comprises at least two monomer domains connected by a linker; each monomer domain exhibits a binding specificity for a target molecule; and each monomer domain is a non-naturally occurring monomer domain.

The present invention also provides polypeptides comprising at least two monomer domains separated by a heterologous linker sequence. In some embodiments, each monomer domain specifically binds to a target molecule; and each monomer domain is a non-naturally occurring protein monomer domain.

Further described herein are polypeptides comprising a first monomer domain that binds a first target molecule and a second monomer domain that binds a second target molecule. The polypeptides may comprise two monomer domains, each monomer domain having a binding specificity that is specific for a different site on the same target molecule. The polypeptides may further comprise a monomer domain having a binding specificity for a second target molecule.

As further described herein, the monomer domains of a library, multimer or polypeptide may be at least 70% identical.

### DEFINITIONS

Unless otherwise indicated, the following definitions supplant those in the art.

The term "monomer domain" or "monomer" is used interchangeably herein refer to a discrete region found in a protein or polypeptide. A monomer domain forms a native three-dimensional structure in solution in the absence of flanking native amino acid sequences. Monomer domains of the invention will specifically bind to a target molecule. For example, a polypeptide that forms a three-dimensional structure that binds to a target molecule is a monomer domain. As used herein, the term "monomer domain" does not encompass the complementarity determining region (CDR) of an antibody.

The term "monomer domain variant" refers to a domain resulting from human-manipulation of a monomer domain sequence. Examples of man-manipulated changes include, e.g., random mutagenesis, site-specific mutagenesis, shuffling, directed evolution, etc. The term "monomer domain variant" does not embrace a mutagenized complementarity determining region (CDR) of an antibody.

The term "multimer" is used herein to indicate a polypeptide comprising at least two monomer domains and/or immuno-domains (e.g., at least two monomer domains, at least two immuno-domains, or at least one monomer domain and at least one immuno-domain). The separate monomer domains and/or immuno-domains in a multimer can be joined together by a linker. A multimer is also known as a combinatorial mosaic protein or a recombinant mosaic protein.

The term "ligand," also referred to herein as a "target molecule," encompasses a wide variety of substances and molecules, which range from simple molecules to complex targets. Target molecules can be proteins, nucleic acids, lipids, carbohydrates or any other molecule capable of recognition by a polypeptide domain. For example, a target molecule can include a chemical compound (i.e., non-biological compound such as, e.g., an organic molecule, an inorganic molecule, or a molecule having both organic and inorganic atoms, but excluding polynucleotides and proteins), a mixture of chemical compounds, an array of spatially localized compounds, a biological macromolecule, a bacteriophage peptide display library, a polysome peptide display library, an extract made from a biological materials such as bacteria, plants, fungi, or animal (e.g., mammalian) cells or tissue, a protein, a toxin, a peptide hormone, a cell, a virus, or the like. Other target molecules include, e.g., a whole cell, a whole tissue, a mixture of related or unrelated proteins, a mixture of viruses or bacterial strains or the like. Target molecules can also be defined by inclusion in screening assays described herein or by enhancing or inhibiting a specific protein interaction (i.e., an agent that selectively inhibits a binding interaction between two predetermined polypeptides).

As used herein, the term "immuno-domains" refers to protein binding domains that contain at least one complementarity determining region (CDR) of an antibody. Immuno-domains can be naturally occurring immunological domains (i.e. isolated from nature) or can be non-naturally occurring immunological domains that have been altered by human-manipulation (e.g., via mutagenesis methods, such as, for example, random mutagenesis, site-specific mutagenesis, and the like, as well as by directed evolution methods, such as, for example, recursive error-prone PCR, recursive recombination, and the like.). Different types of immuno-domains that are suitable for use in the practice of the present invention include a minibody, a single-domain antibody, a single chain variable fragment (ScFv), and a Fab fragment.

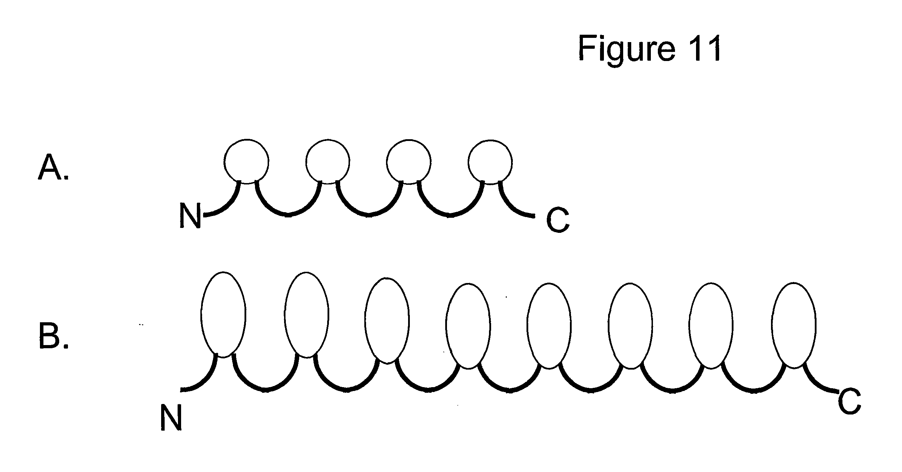

The term "minibody" refers herein to a polypeptide that encodes only 2 complementarity determining regions (CDRs) of a naturally or non-naturally (e.g., mutagenized) occurring heavy chain variable domain or light chain variable domain, or combination thereof. An example of a minibody is described by Pessi et al., A designed metal-binding protein with a novel fold, (1993) Nature 362:367-369. A multimer of minibodies is schematically illustrated in Figure 11A. The circles depict minibodies, and the solid lines depict the linker moieties joining the immuno-domains to each other.

As used herein, the term "single-domain antibody" refers to the heavy chain variable domain ("V_{H}") of an antibody, i.e., a heavy chain variable domain without a light chain variable domain. Exemplary single-domain antibodies employed in the practice of the present invention include, for example, the Camelid heavy chain variable domain (about 118 to 136 amino acid residues) as described in Hamers-Casterman, C. et al., Naturally occurring antibodies devoid of light chains (1993) Nature 363:446-448, and Dumoulin, et al., Single-domain antibody fragments with high conformational stability (2002) Protein Science 11:500-515. A multimer of single-domain antibodies is depicted in Figure 11B. The ellipses represent the single-domain antibodies, and the solid lines depict the linker moieties joining the single-domain antibodies to each other.

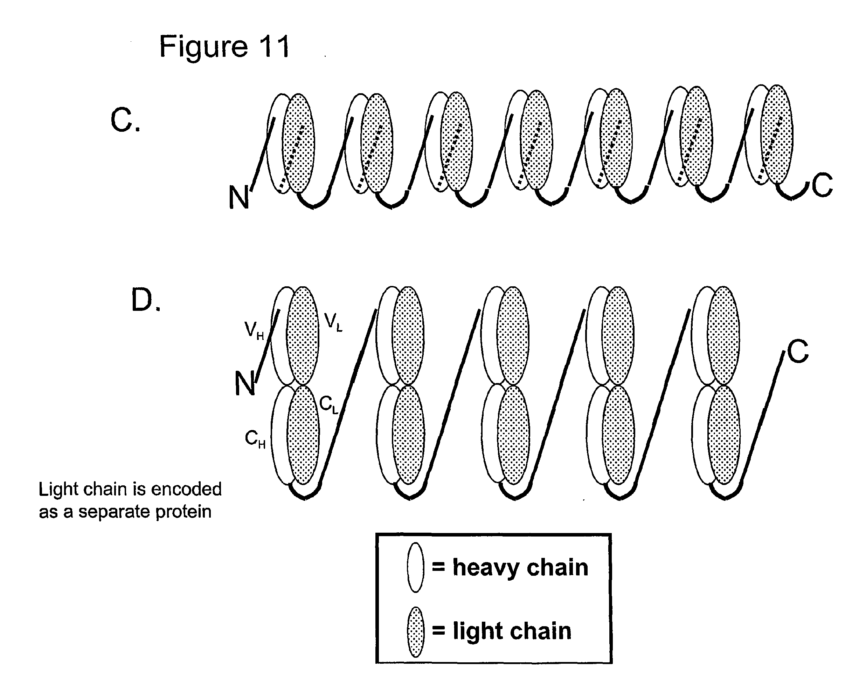

The terms "single chain variable fragment" or "ScFv" are used interchangeably herein to refer to antibody heavy and light chain variable domains that are joined by a peptide linker having at least 12 amino acid residues. Single chain variable fragments contemplated for use in the practice of the present invention include those described in Bird, et al., Single-chain antigen-binding proteins (1988) Science 242(4877):423-426 and Huston et al., Protein engineering of antibody binding sites: recovery of specific activity in an anti-digoxin single-chain Fv analogue produced in Escherichia coli (1988) Proc Natl Acad Sci U S A 85(16):5879-83. A multimer of single chain variable fragments is illustrated in Figure 11C. The dotted lines represent the peptide linker joining the heavy and light chain variable domains to each other. The solid lines depict the linker moieties joining the heavy chain variable domains to each other.

As used herein, the term "Fab fragment" refers to an immuno-domain that has two protein chains, one of which is a light chain consisting of two light chain domains (V_{L} variable domain and C_{L} constant domain) and a heavy chain consisting of two heavy domains (i.e., a V_{H} variable and a C_{H} constant domain). Fab fragments employed in the practice of the present invention include those that have an interchain disulfide bond at the C-terminus of each heavy and light component, as well as those that do not have such a C-terminal disulfide bond. Each fragment is about 47 kD. Fab fragments are described by Pluckthun and Skerra, Expression of functional antibody Fv and Fab fragments in Escherichia col (1989) Methods Enzymol 178:497-515. A multimer of Fab fragments is depicted in Figure 11D. The white ellipses represent the heavy chain component of the Fab fragment, the filled ellipses represent the light chain component of the Fab.

The term "linker" is used herein to indicate a moiety or group of moieties that joins or connects two or more discrete separate monomer domains. The linker allows the discrete separate monomer domains to remain separate when joined together in a multimer. The linker moiety is typically a substantially linear moiety. Suitable linkers include polypeptides, polynucleic acids, peptide nucleic acids and the like. Suitable linkers also include optionally substituted alkylene moieties that have one or more oxygen atoms incorporated in the carbon backbone. Typically, the molecular weight of the linker is less than about 2000 daltons. More typically, the molecular weight of the linker is less than about 1500 daltons and usually is less than about 1000 daltons. The linker can be small enough to allow the discrete separate monomer domains to cooperate, e.g., where each of the discrete separate monomer domains in a multimer binds to the same target molecule via separate binding sites. Exemplary linkers include a polynucleotide encoding a polypeptide, or a polypeptide of amino acids or other non-naturally occurring moieties. The linker can be a portion of a native sequence, a variant thereof, or a synthetic sequence. Linkers can comprise, e.g., naturally occurring, non-naturally occurring amino acids, or a combination of both.

The term "separate" is used herein to indicate a property of a moiety that is independent and remains independent even when complexed with other moieties, including for example, other monomer domains. A monomer domain is a separate domain in a protein because it has an independent property that can be recognized and separated from the protein. For instance, the ligand binding ability of the A-domain in the LDLR is an independent property. Other examples of separate include the separate monomer domains in a multimer that remain separate independent domains even when complexed or joined together in the multimer by a linker. Another example of a separate property is the separate binding sites in a multimer for a ligand.

As used herein, "directed evolution" refers to a process by which polynucleotide variants are generated, expressed, and screened for an activity (e.g., a polypeptide with binding activity) in a recursive process. One or more candidates in the screen are selected and the process is then repeated using polynucleotides that encode the selected candidates to generate new variants. Directed evolution involves at least two rounds of variation generation and can include 3, 4, 5, 10, 20 or more rounds of variation generation and selection. Variation can be generated by any method known to those of skill in the art, including, e.g., by error-prone PCR, gene shuffling, chemical mutagenesis and the like.

The term "shuffling" is used herein to indicate recombination between non-identical sequences. In some embodiments, shuffling can include crossover via homologous recombination or via non-homologous recombination, such as via cre/lox and/or flp/frt systems. Shuffling can be carried out by employing a variety of different formats, including for example, *in vitro* and *in vivo* shuffling formats, in silico shuffling formats, shuffling formats that utilize either double-stranded or single-stranded templates, primer based shuffling formats, nucleic acid fragmentation-based shuffling formats, and oligonucleotide-mediated shuffling formats, all of which are based on recombination events between non-identical sequences and are described in more detail or referenced herein below, as well as other similar recombination-based formats.

The term "random" as used herein refers to a polynucleotide sequence or an amino acid sequence composed of two or more amino acids and constructed by a stochastic or random process. The random polynucleotide sequence or amino acid sequence can include framework or scaffolding motifs, which can comprise invariant sequences.

The term "pseudorandom" as used herein refers to a set of sequences, polynucleotide or polypeptide, that have limited variability, so that the degree of residue variability at some positions is limited, but any pseudorandom position is allowed at least some degree of residue variation.

The terms "polypeptide," "peptide," and "protein" are used herein interchangeably to refer to an amino acid sequence of two or more amino acids.

'Conservative amino acid substitution" refers to the interchangeability of residues having similar side chains. For example, a group of amino acids having aliphatic side chains is glycine, alanine, valine, leucine, and isoleucine; a group of amino acids having aliphatic-hydroxyl side chains is serine and threonine; a group of amino acids having amide-containing side chains is asparagine and glutamine; a group of amino acids having aromatic side chains is phenylalanine, tyrosine, and tryptophan; a group of amino acids having basic side chains is lysine, arginine, and histidine; and a group of amino acids having sulfur-containing side chains is cysteine and methionine. Preferred conservative amino acids substitution groups are: valine-leucine-isoleucine, phenylalanine-tyrosine, lysine-arginine, alanine-valine, and asparagine-glutamine.

The phrase "nucleic acid sequence" refers to a single or double-stranded polymer of deoxyribonucleotide or ribonucleotide bases read from the 5' to the 3' end. It includes chromosomal DNA, self-replicating plasmids and DNA or RNA that performs a primarily structural role.

The term "encoding" refers to a polynucleotide sequence encoding one or more amino acids. The term does not require a start or stop codon. An amino acid sequence can be encoded in any one of six different reading frames provided by a polynucleotide sequence.

The term "promoter" refers to regions or sequence located upstream and/or downstream from the start of transcription that are involved in recognition and binding of RNA polymerase and other proteins to initiate transcription.

A "vector" refers to a polynucleotide, which when independent of the host chromosome, is capable of replication in a host organism. Examples of vectors include plasmids. Vectors typically have an origin of replication. Vectors can comprise, e.g., transcription and translation terminators, transcription and translation initiation sequences, and promoters useful for regulation of the expression of the particular nucleic acid.

The term "recombinant" when used with reference, *e*.*g*., to a cell, or nucleic acid, protein, or vector, indicates that the cell, nucleic acid, protein or vector, has been modified by the introduction of a heterologous nucleic acid or protein or the alteration of a native nucleic acid or protein, or that the cell is derived from a cell so modified. Thus, for example, recombinant cells express genes that are not found within the native (nonrecombinant) form of the cell or express native genes that are otherwise abnormally expressed, under-expressed or not expressed at all.

The phrase "specifically (or selectively) binds" to a polypeptide, when referring to a monomer or multimer, refers to a binding reaction that can be determinative of the presence of the polypeptide in a heterogeneous population of proteins and other biologics. Thus, under standard conditions or assays used in antibody binding assays, the specified monomer or multimer binds to a particular target molecule above background (e.g., 2X, 5X, 10X or more above background) and does not bind in a significant amount to other molecules present in the sample.

The terms "identical" or percent "identity," in the context of two or more nucleic acids or polypeptide sequences, refer to two or more sequences or subsequences that are the same. "Substantially identical" refers to two or more nucleic acids or polypeptide sequences having a specified percentage of amino acid residues or nucleotides that are the same (*i.e.,* 60% identity, optionally 65%, 70%, 75%, 80%, 85%, 90%, or 95% identity over a specified region, or, when not specified, over the entire sequence), when compared and aligned for maximum correspondence over a comparison window, or designated region as measured using one of the following sequence comparison algorithms or by manual alignment and visual inspection. Optionally, the identity or substantial identity exists over a region that is at least about 50 nucleotides in length, or more preferably over a region that is 100 to 500 or 1000 or more nucleotides or amino acids in length.

The term "heterologous linker," when used in reference to a multimer, indicates that the multimer comprises a linker and a monomer that are not found in the same relationship to each other in nature (*e.g.*, they form a fusion protein).

### BRIEF DESCRIPTION OF THE DRAWINGS

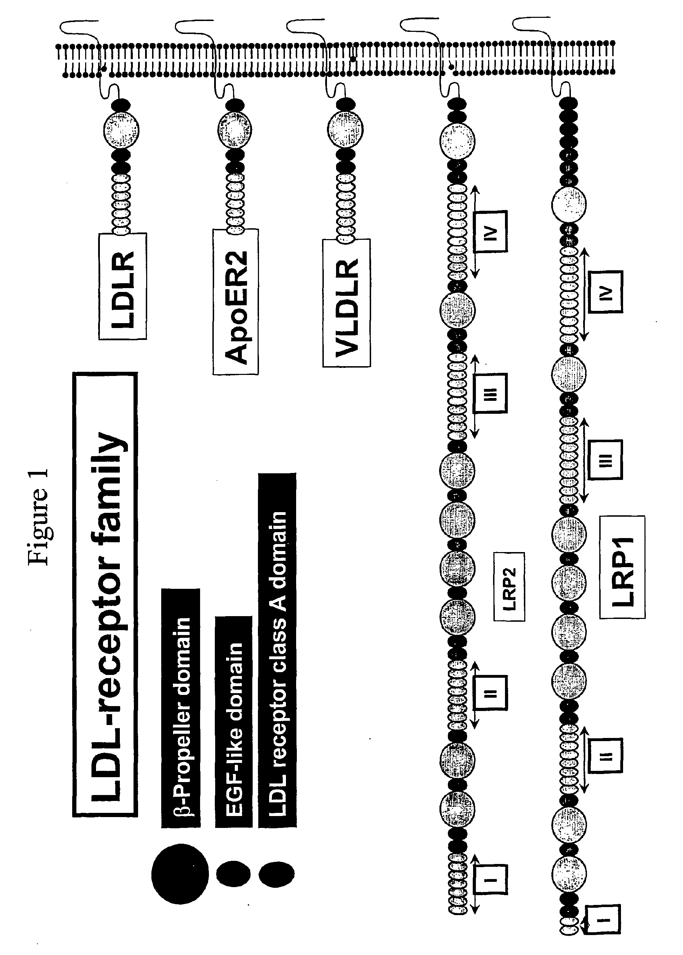

Figure 1 schematically illustrates the type, number and order of monomer domains found in members of the LDL-receptor family. These monomer domains include β-Propeller domains, EGF-like domains and LDL receptor class A-domains. The members shown include low-density lipoprotein receptor (LDLR), ApoE Receptor 2 (ApoER2), very-low-density lipoprotein receptor (VLDLR), LDLR-related protein 2 (LRP2) and LDLR-related protein1 (LRP1).

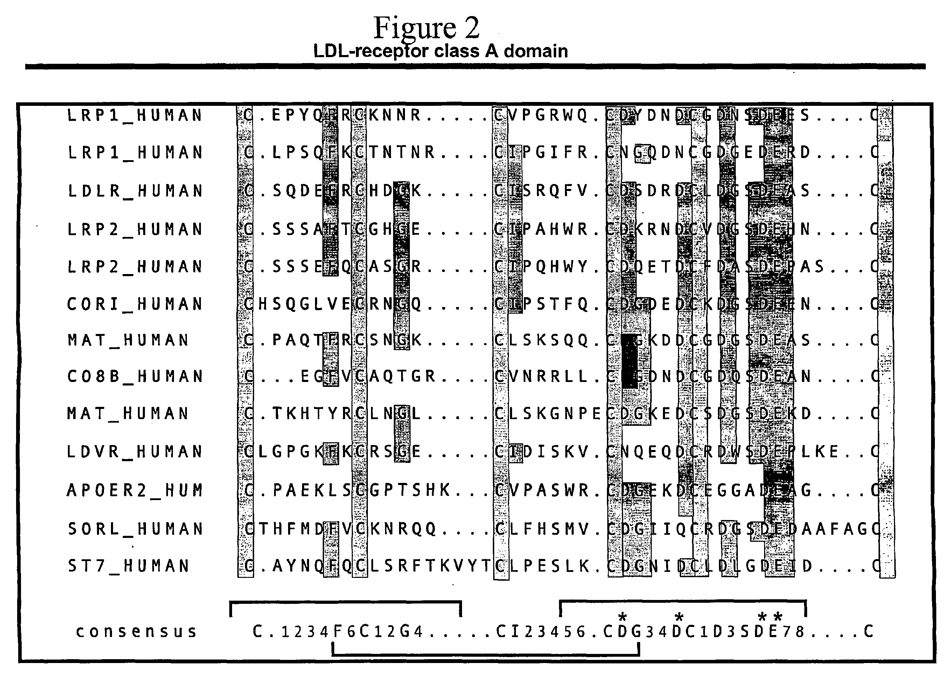

Figure 2 schematically illustrates the alignment of partial amino acid sequence from a variety of the LDL-receptor class A-domains that include two human LRP1 sequences, two human LRP2 sequences, two human LDLR sequences, two human LDVR sequences, one human LRP3 sequence, one human MAT sequence, a human CO6 sequence, and a human SORL sequence, to demonstrate the conserved cysteines.

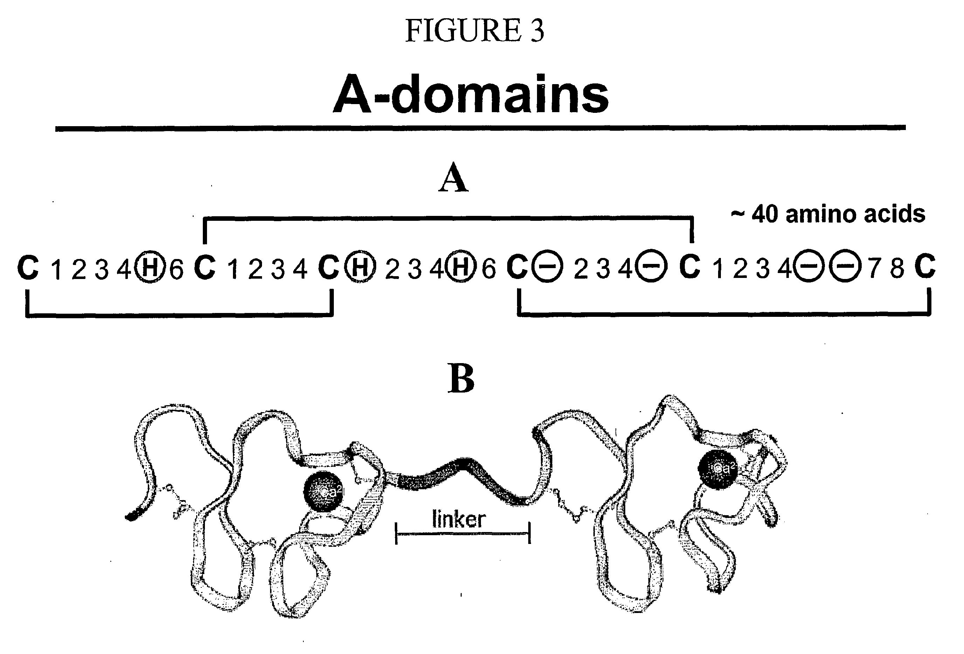

Figure 3, panel A schematically illustrates an example of an A-domain. Panel A schematically illustrates conserved amino acids in an A-domain of about 40 amino acids long. The conserved cysteine residues are indicated by C, and the negatively charged amino acids are indicated by a circle with a minus ("-") sign. Circles with an "H" indicate hydrophobic residues. Panel B schematically illustrates two folded A-domains connected via a linker. Panel B also indicates two calcium binding sites, dark circles with Ca⁺², and three disulfide bonds within each folded A-domain for a total of 6 disulfide bonds.

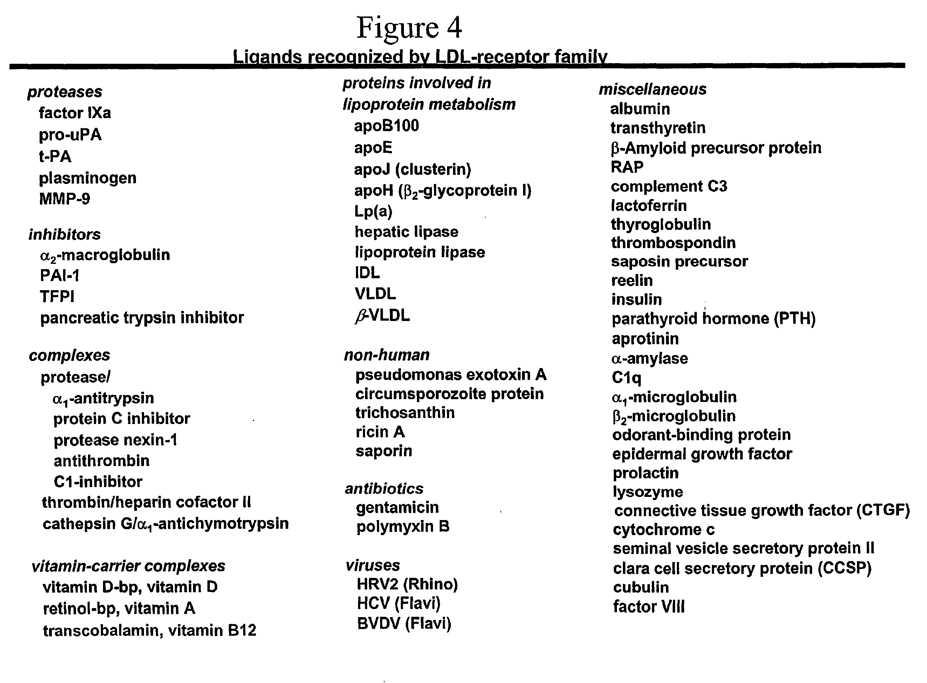

Figure 4 indicates some of the ligands recognized by the LDL-receptor family, which include inhibitors, proteases, protease complexes, vitamin-carrier complexes, proteins involved in lipoprotein metabolism, non-human ligands, antibiotics, viruses, and others.

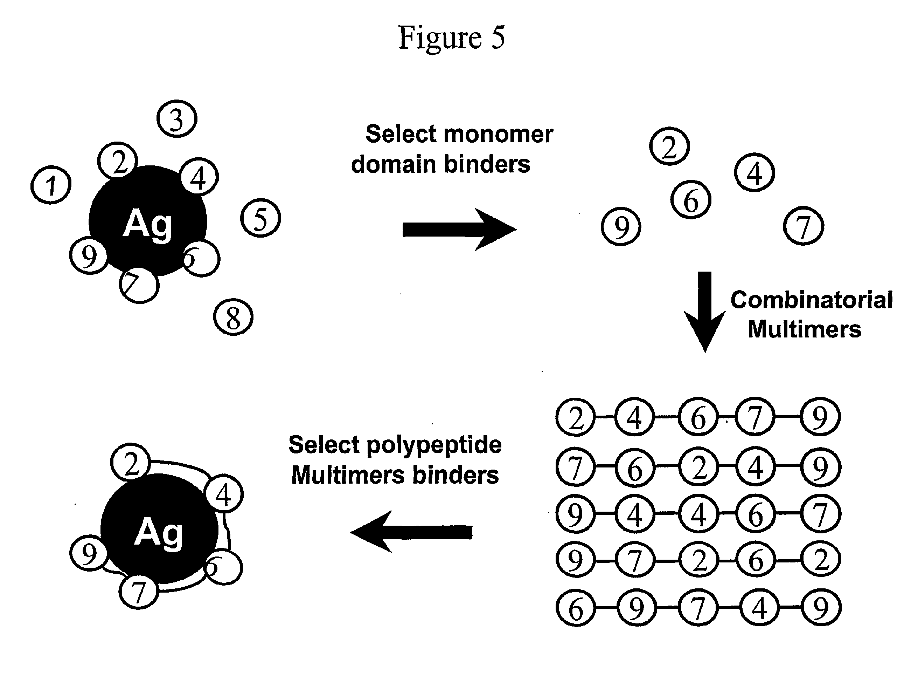

Figure 5 schematically illustrates a general scheme for identifying monomer domains that bind to a ligand, isolating the selected monomer domains, creating multimers of the selected monomer domains by joining the selected monomer domains in various combinations and screening the multimers to identify multimers comprising more than one monomer that binds to a ligand.

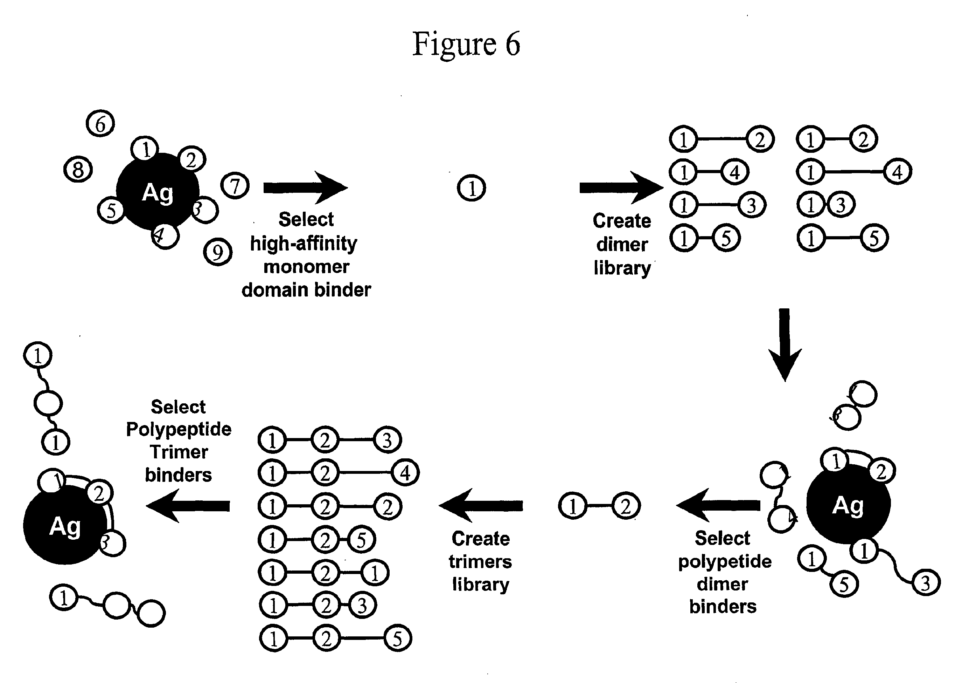

Figure 6 is a schematic representation of another selection strategy (guided selection). A monomer domain with appropriate binding properties is identified from a library of monomer domains. The identified monomer domain is then linked to monomer domains from another library of monomer domains to form a library of multimers. The multimer library is screened to identify a pair of monomer domains that bind simultaneously to the target. This process can then be repeated until the optimal binding properties are obtained in the multimer.

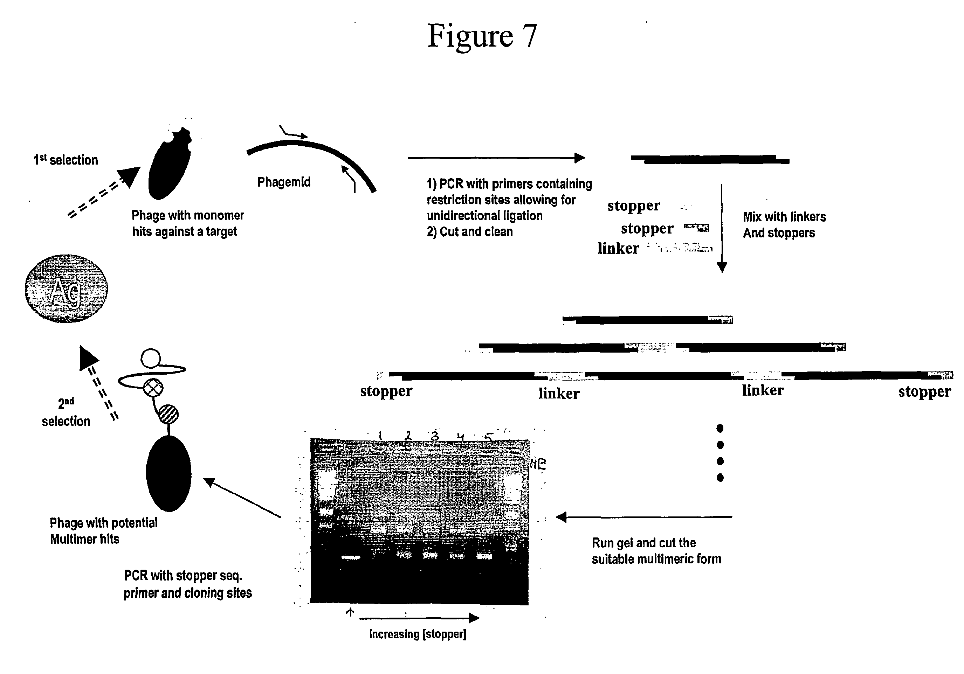

Figure 7 shows the multimerization process of monomer domains. The target-binding monomer hits are amplified from a vector. This mixture of target-binding monomer domains and/or immuno-domains is then cleaved and mixed with an optimal combination of linker and stopper oligonucleotides. The multimers that are generated are then cloned into a suitable vector for the second selection step for identification of target-binding multimers.

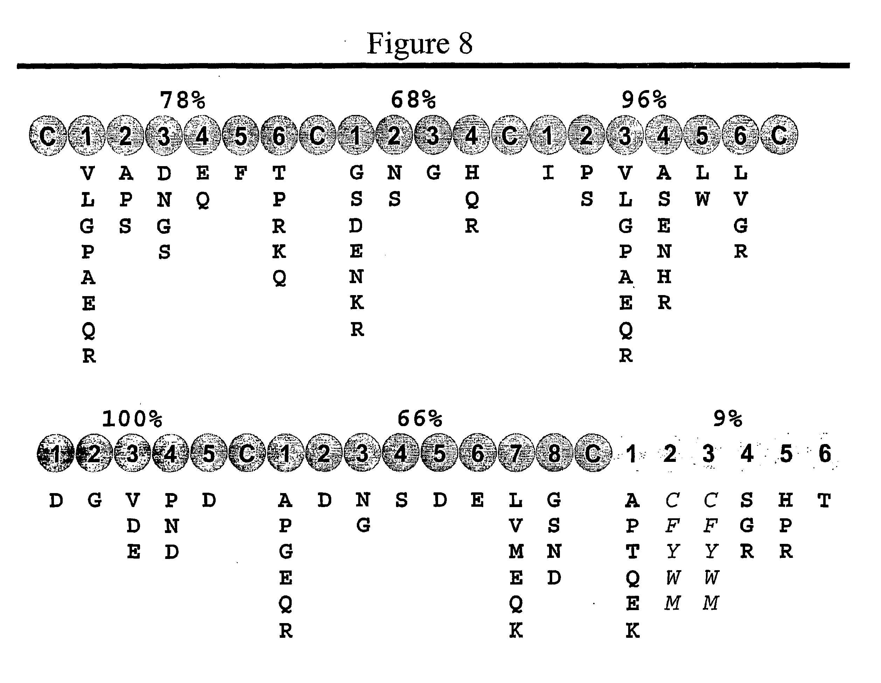

Figure 8 depicts common amino acids in each position of the A domain. The percentages above the amino acid positions refer to the percentage of naturally-occurring A domains with the inter-cysteine spacing displayed. Potential amino acid residues in bold depicted under each amino acid position represent common residues at that position. The final six amino acids, depicted as lighter-colored circles, represent linker sequences. The two columns of italicized amino acid residues at positions 2 and 3 of the linker represent amino acid residues that do not occur at that position. Any other amino acid (e.g., A, D, E, G, H, I, K, L, N, P, Q, R, S, T, and V) may be included at these positions.

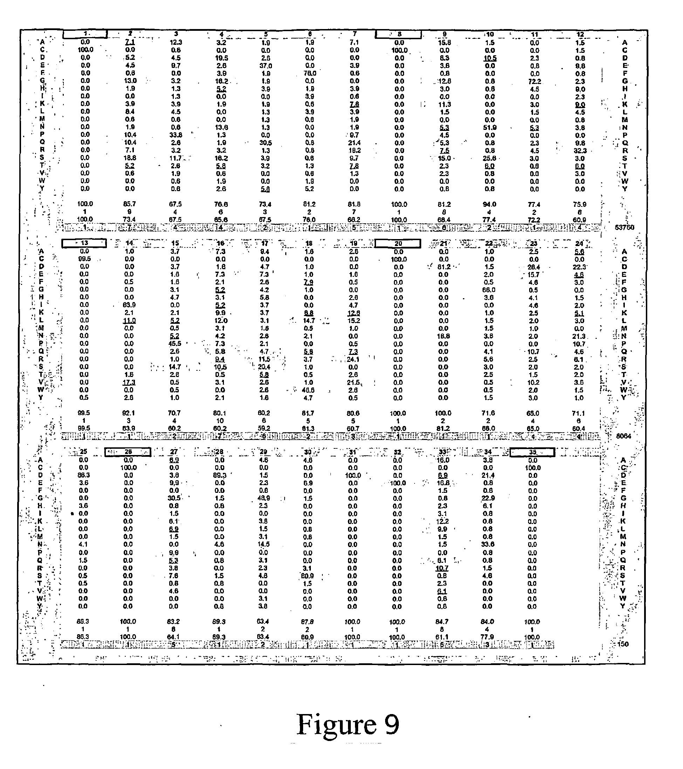

Figure 9 displays the frequency of occurrence of amino acid residues in naturally-occurring A domains for A domains with the following spacing between cysteines: CX₆CX₄CX₆CX₅CX₈C.

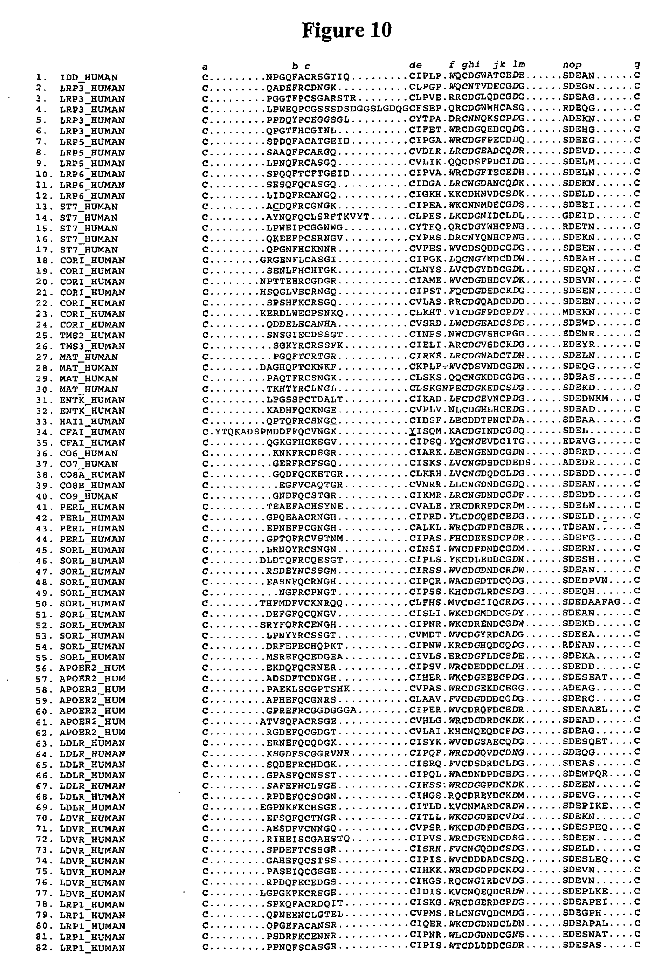

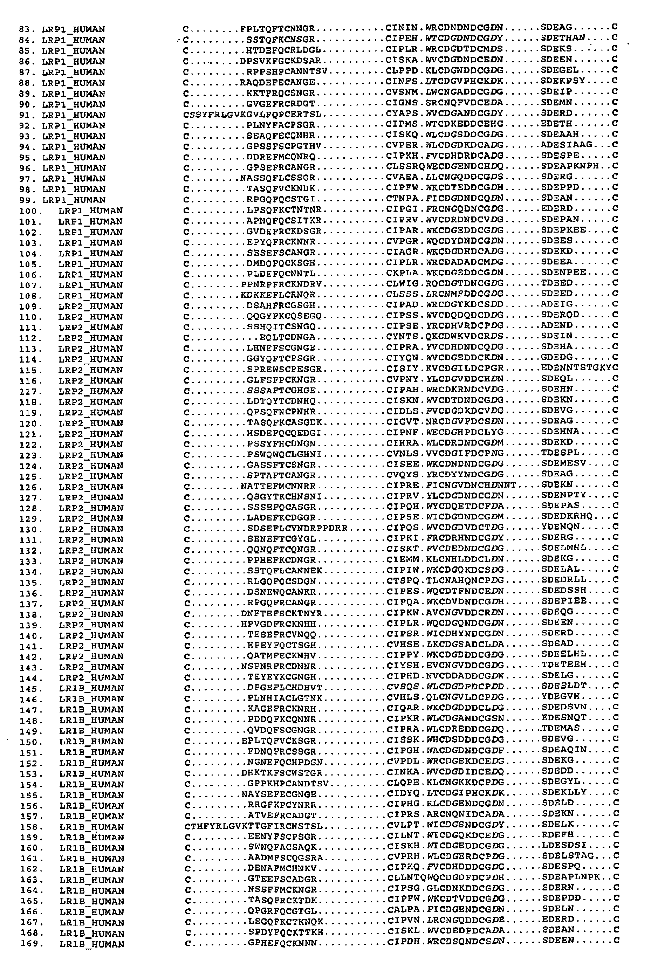

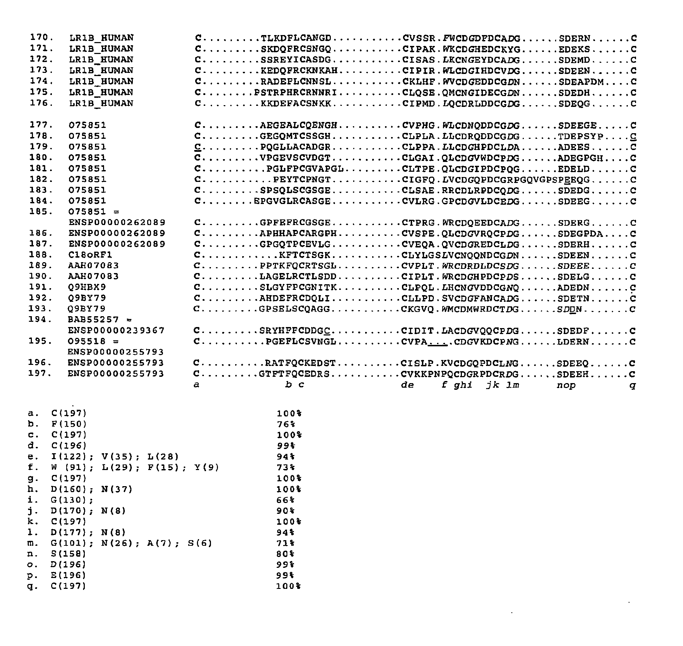

Figure 10 depicts an alignment of A domains. At the top and the bottom of the figure, small letters (a-q) indicate conserved residues. The predominant amino acids at these positions and the percent of time they were observed in native A domains is illustrated at the bottom of the figure.

Figure 11 depicts possible multimer conformations comprises of immuno-domains. Figure 11A illustrates a multimer of minibodies. Figure 11B illustrates a multimer of single-domain antibodies. Figure 11C illustrates a immuno-domain multimer of scfvs. Figure 11D illustrates a multimer of Fab fragments.

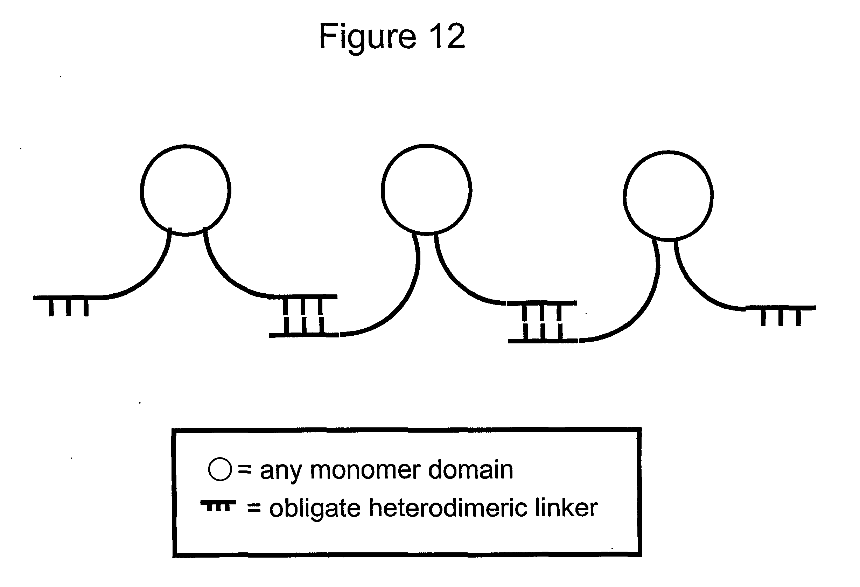

Figure 12 depicts linkage of domains via partial linkers.

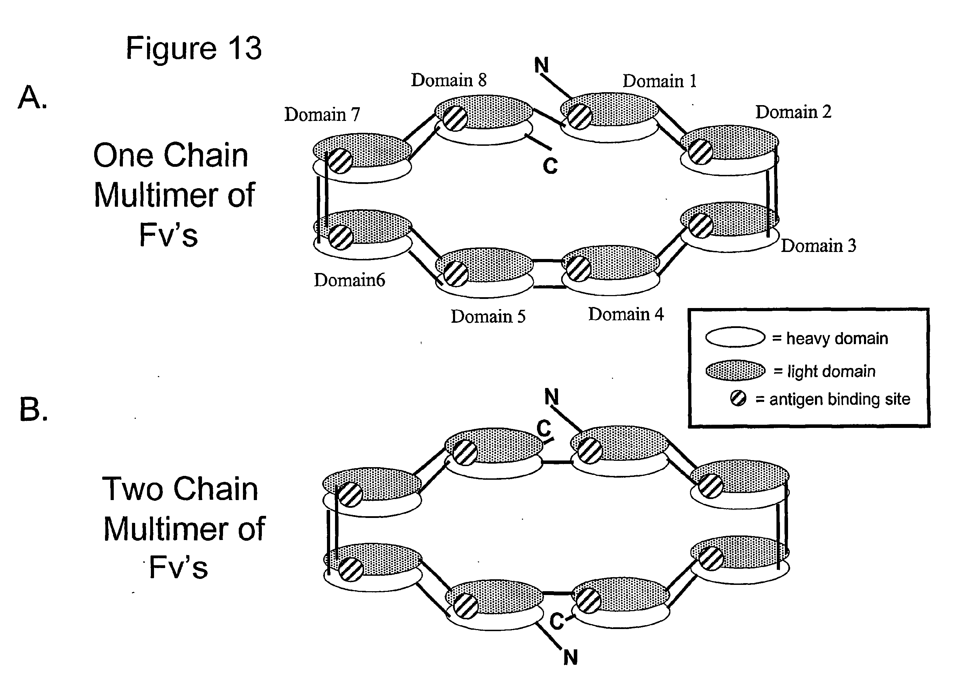

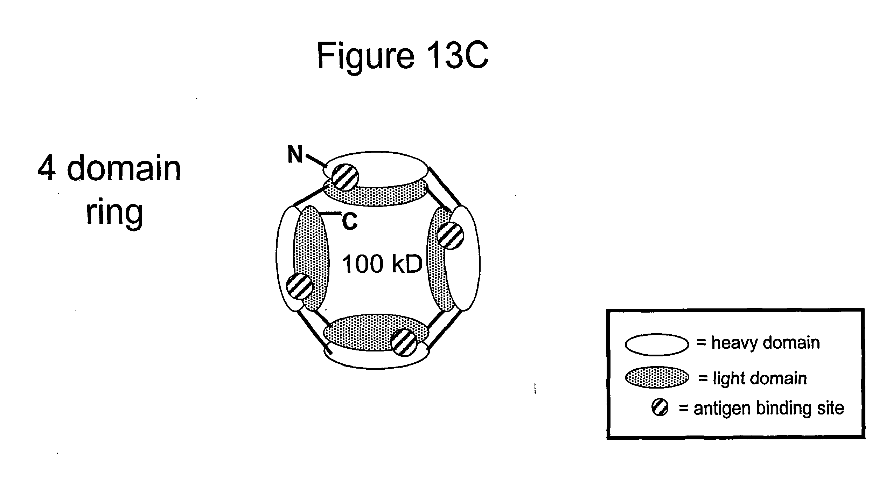

Figure 13 illustrates exemplary multimer ring formations.

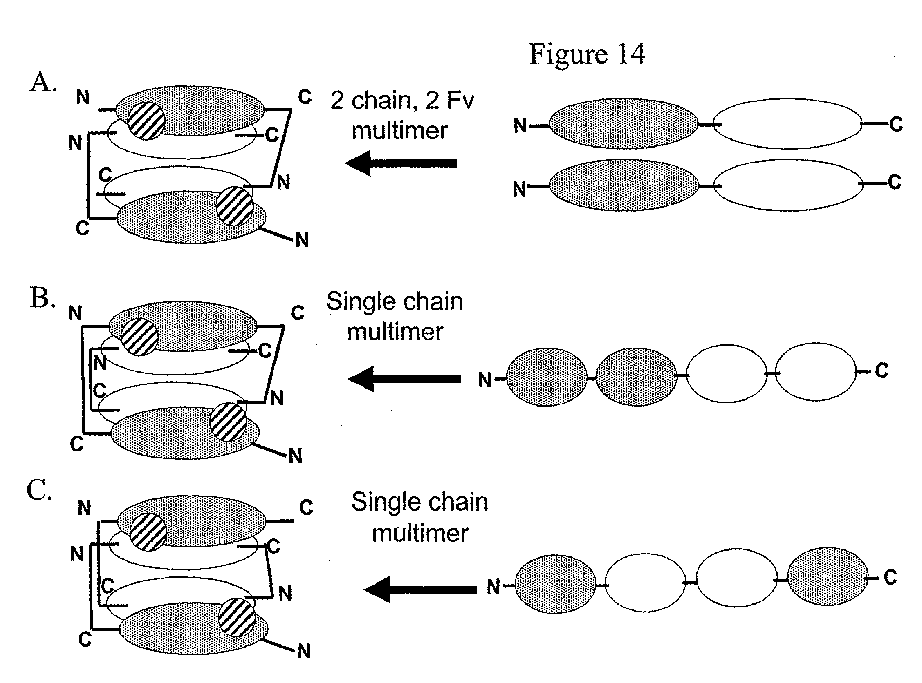

Figure 14 illustrates various multimer conformations of heavy and light chains of Fvs.

### DETAILED DESCRIPTION OF THE INVENTION

The invention provides an enhanced approach for selecting and optimizing properties of discrete LDL receptor class A monomer domains to create multimers. In particular, this disclosure describes methods, compositions and kits for identifying discrete LDL receptor class A monomer domains that bind to a desired ligand or mixture of ligands and creating multimers (also known as combinatorial mosaic proteins or recombinant mosaic proteins) that comprise two or more monomer domains and/or immuno-domains that are joined via a linker. The multimers can be screened to identify those that have an improved phenotype such as improved avidity or affinity or altered specificity for the ligand or the mixture of ligands, compared to the discrete monomer domain.

### 1. Discrete Monomer Domains

Monomer domains can be polypeptide chains with about 30 to about 100 amino acids. Similarly, a monomer domain of the present invention comprises 30 to about 100 amino acids. Monomer domains and immuno-domains can maintain a stable conformation in solution. The stable conformation contains disulfide bonds (e.g., at least two, or three or more disulfide bonds). The disulfide bonds can optionally be formed between two cysteine residues.

Publications describing monomer domains and mosaic proteins and references cited within include the following: Hegyi, H and Bork, P., On the classification and evolution ofprotein modules, (1997) J. Protein Chem., 16(5):545-551; Baron et al., Protein modules (1991) Trends Biochem. Sci., 16(1):13-7; Ponting et al., Evolution of domain families, (2000), Adv. Protein Chem., 54:185-244; Doolittle, The multiplicity of domains in proteins, (1995) Annu. Rev. Biochem 64:287-314; Doolitte and Bork, Evolutionarily mobile modules in proteins (1993) Scientific American, 269 (4):50-6; and Bork, Shuffled domains in extracellular proteins (1991), FEBS letters 286(1-2):47-54. Monomer domains of the present invention also include those domains found in Pfam database and the SMART database. *See* Schultz, et al., SMART: a web-based tool for the study of genetically mobile domains, (2000) Nucleic Acid Res. 28(1):231-34.

Monomer domains suitable for use in the practice of the present invention are LDL-receptor class A domain, Figure 1 schematically diagrams various kinds of monomer domains found in molecules in the LDL-receptor family.

Other features of monomer domains include the ability to bind ligands (e.g., as in the LDL receptor class A domain, the ability to bind an ion (e.g., Ca²⁺ binding by the LDL receptor A-domain).

Characteristics of a monomer domain include the ability to fold independently and the ability to form a stable structure. Thus, the structure of the monomer domain is often conserved, although the polynucleotide sequence encoding the monomer need not be conserved. For example, the A-domain structure is conserved among the members of the A-domain family, while the A-domain nucleic acid sequence is not. Thus, for example, a monomer domain is classified as an A-domain by its cysteine residues and its affinity for calcium, not necessarily by its nucleic acid sequence. *See,* Figure 2.

Specifically, the A-domains (sometimes called "complement-type repeats") contain about 30-50 amino acids. In some embodiments, the domains comprise about 35-45 amino acids and in some cases about 40 amino acids. Within the 30-50 amino acids, there are about 6 cysteine residues. Of the six cysteines, disulfide bonds typically are found between the following cysteines: C1 and C3, C2 and C5, C4 and C6. The A domain constitutes a ligand binding moiety. The cysteine residues of the domain are disulfide linked to form a compact, stable, functionally independent moiety. *See,* Figure 3. Clusters of these repeats make up a ligand binding domain, and differential clustering can impart specificity with respect to the ligand binding.

Exemplary A domain sequences and consensus sequences are depicted in Figures 2, 3 and 8. Figure 9 displays location and occurrence of residues in A domains with the following spacing between cysteines. In addition, Figure 10 depicts a number of A domains and provides a listing of conserved amino acids. One typical consensus sequence useful to identify A domains is the following: C-[VILMA]-X₍₅₎-C-[DNH]-X₍₃₎-[DENQHT]-C-X_{(3,4)}-[STADE]- [DEH]-[DE]-X_{(1,5)}-C, where the residues in brackets indicate possible residues at one position. "X_{(#)}" indicates number of residues. These residues can be any amino acid residue. Parentheticals containing two numbers refers to the range of amino acids that can occupy that position (e.g., "[DE]-X_{(1,5)}-C" means that the amino acids DE are followed by 1, 2, 3, 4, or 5 residues, followed by C). This consensus sequence only represents the portion of the A domain beginning at the third cysteine. A second consensus is as follows: C-X₍₃₋₁₅₎-C-X₍₄₋₁₅₎-C-X₍₆₋₇₎-C-[N,D]-X₍₃₎-[D,E,N,Q,H,S,T]-C-X_{(4,6)}-D-E-X₍₂₋₈₎-C. The second consensus predicts amino acid residues spanning all six cysteine residues. In some embodiments, A domain variants comprise sequences substantially identical to any of the above-described sequences.

To date, at least 190 human A-domains are identified based on cDNA sequences. *See, e.g*., Figure 10. Exemplary proteins containing A-domains include, e.g., complement components (e.g., C6, C7, C8, C9, and Factor I), serine proteases (e.g., enteropeptidase, matriptase, and corin), transmembrane proteins (e.g., ST7, LRP3, LRP5 and LRP6) and endocytic receptors (e.g., Sortilin-related receptor, LDL-receptor, VLDLR, LRP1, LRP2, and ApoER2). A domains and A domain variants can be readily employed in the practice of the present invention as monomer domains and variants thereof. Further description of A domains can be found in the following publications and references cited therein: Howell and Hertz, The LDL receptor gene family: signaling functions during development, (2001) Current Opinion in Neurobiology 11:74-81; Herz (2001), *supra;* Krieger, The "best" of cholesterols, the "worst" of cholesterols: A tale of two receptors, (1998) PNAS 95: 4077-4080; Goldstein and Brown, The Cholesterol Quartet, (2001) Science, 292: 1310-1312; and, Moestrup and Verroust, Megalin-and Cubilin-Mediated Endocytosis ofProtein-Bound Vitamins, Lipids, and Hormones in Polarized Epithelia, (2001) Ann. Rev. Nutr. 21:407-28.

Descriptions of some further monomer domains can be found in the following publications and the references cited therein: Yamazaki et al., Elements of Neural Adhesion Molecules and a Yeast Vacuolar Protein Sorting Receptor are Present in a Novel Mammalian Low Density Lipoprotein Receptor Family Member, (1996) Journal of Biological Chemistry 271(40) 24761-24768; Nakayama et al., Identification of High-Molecular-Weight Proteins with Multiple EGF-like Motifs by Motif-Trap Screening, (1998) Genomics 51:27-34; Liu et al, Genomic Organization ofNew Candidate Tumor Suppressor Gene, LRP1B, (2000) Genomics 69:271-274; Liu et al., The Putative Tumor Suppressor LRP1B, a Novel Member of the Low Density Lipoprotein (LDL) Receptor Family, Exhibits Both Overlapping and Distinct Properties with the LDL Receptor-related Protein, (2001) Journal of Biological Chemistry 276(31):28889-28896; Ishii et al, cDNA of a New Low-Density Lipoprotein Receptor-Related Protein and Mapping of its Gene (LRP3) to Chromosome Bands 19q12-q13.2, (1998) Genomics 51:132-135; Orlando et al, Identification of the second cluster of ligand binding repeats in megalin as a site for receptor-ligand interactions, (1997) PNAS USA 94:2368-2373; Jeon and Shipley, Vesicle-reconstituted Low Density Lipoprotein Receptor, (2000) Journal of Biological Chemistry 275(39):30458-30464; Simmons et al., Human Low Density Lipoprotein Receptor Fragment, (1997) Journal of Biological Chemistry 272(41):25531-25536; Fass et al., Molecular Basis of familial hypercholesterolaemia from structure of LDL receptor module, (1997) Nature 388:691-93; Daly et al., Three-dimensional structure of a cysteine-rich repeat from the low-density lipoprotein receptor, (1995) PNAS USA 92:6334-6338; North and Blacklow, Structural Independence of Ligand-Binding Modules Five and Six of the LDL Receptor, (1999) Biochemistry 38:3926-3935; North and Blacklow, Solution Structure of the Sixth LDL-A module of the LDL Receptor, (2000) Biochemistry 39:25640-2571; North and Blacklow, Evidence that Familial Hypercholesterolemia Mutations of the LDL Receptor Cause Limited Local Misfolding in an LDL-A Module Pair, (2000) Biochemistry 39:13127-13135; Beglova et al., Backbone Dynamics of a Module Pair from the Ligand-Binding Domain of the LDL Receptor, (2001) Biochemistry 40:2808-2815; Bieri et al., Folding, Calcium binding, and Structural Characterization of a Concatemer of the First and Second Ligand-Binding Modules of the Low-Density Lipoprotein Receptor, (1998) Biochemistry 37:10994-11002; Jeon et al., Implications for familial hypercholesterolemia from the structure of the LDL receptor YWTD-EGF domain pair, (2001) Nature Structural Biology 8(6):499-504; Kurniawan et al., NMR structure of a concatemer of the first and second ligand-binding modules of the human low-density lipoprotein receptor, (2000) Protein Science 9:1282-1293; Esser et al., Mutational Analysis of the Ligand Binding Domain of the Low Density poprotein Receptor, (1988) Journal of Biological Chemistry 263(26):13282-13290; Russell et al., Different Combinations of Cysteine-rich Repeats Mediate Binding of Low Density Lipoprotein Receptor to Two Different Proteins, (1989) Journal of Biological Chemistry 264(36):21682-21688; Davis et al., Acid-dependent ligand dissociation and recycling of LDL receptor mediated by growth factor homology region, (1987) Nature 326:760-765; Rong et al., Conversion of a human low-density lipoprotein receptor ligand-binding repeat to a virus receptor: Identification of residues important for ligand specificity, (1998) PNAS USA 95:8467-8472; Agnello et al., Hepatitis C virus and other Flaviviridae viruses enter cells via low density lipoprotein receptor; (1999) PNAS 96(22):12766-12771; Esser and Russell, Transport-deficient Mutations in the Low Density lipoprotein receptor, (1988) Journal of Biological Chemistry 263(26):13276-13281; Davis et al., The Low Density Lipoprotein Receptor, (1987) Journal of Biological Chemistry 262(9):4075-4082; and, Peacock et al., Human Low Density Lipoprotein Receptor Expressed in Xenopus Oocytes, (1988) Journal of Biological Chemistry 263(16):7838-7845.

Others publications that describe the VLDLR, ApoER2 and LRP1 proteins and their monomer domains include the following as well as the references cited therein: Savonen et al., The Carboxyl-terminal Domain of Receptor-associated Protein Facilitates Proper Folding and Trafficking of the Very Low Density Lipoprotein Receptor by Interaction with the Three Amino-terminal Ligand-binding Repeats of the Receptor, (1999) Journal of Biological Chemistry 274(36):25877-25882; Hewat et al., The cellular receptor to human rhinovirus 2 binds around the 5-fold axis and not in the canyon: a structural view, (2000) EMBO Journal 19(23):6317-6325; Okun et al., VLDL Receptor Fragments of Different Lengths Bind to Human Rhinovirus HRV2 with Different Stoichiometry, (2001) Journal of Biological Chemistry 276(2):1057-1062; Rettenberger et al., Ligand Binding Properties of the Very Low Density Lipoprotein Receptor, (1999) Journal of Biological Chemistry 274(13):8973-8980; Mikhailenko et al., Functional Domains of the very low density lipoprotein receptor: molecular analysis of ligand binding and acid-dependent ligand dissociation mechanisms, (1999) Journal of Cell Science 112:3269-3281; Brandes et al., Alternative Splicing in the Ligand Binding Domain of Mouse ApoE Receptor-2 Produces Receptor Variants Binding Reelin but not alpa2-rnacroglobulin, (2001) Journal of Biological Chemistry 276(25):22160-22169; Kim et al., Exon/Intron Organization, Chromosome Localization, Alternative Splicing, and Transcription Units of the Human Apolipoprotein E Receptor 2 Gene, (1997) Journal of Biological Chemistry 272(13):8498-8504; Obermoeller-McCormick et al., Dissection of receptor folding and ligand-binding property with functional minireceptors of LDL receptor-related protein, (2001) Journal of Cell Science 114(5):899-908; Horn et al., Molecular Analysis of Ligand Binding of the Second Cluster of Complement-type Repeats of the Low Density Lipoprotein Receptor-related Protein, (1997) Journal of Biological Chemistry 272(21):13608-13613; Neels et al., The Second and Fourth Cluster of Class A Cysteine-rich Repeats of the Low Density Lipoprotein Receptor-related Protein Share Ligand binding Properties, (1999) Journal of Biological Chemistry 274(44):31305-31311; Obermoeller et al., Differential Functions of the Triplicated Repeats Suggest Two Independent Roles for the Receptor-Associated Protein as a Molecular Chaperone, (1997) Journal of Biological Chemistry 272(16):10761-10768; Andersen et al., Identification of the Minimal Functional Unit in the Low Density Lipoprotein Receptor-related Protein for Binding the Receptor-associated Protein (RAP), (2000) Journal of Biological Chemistry 275(28):21017-21024; Andersen et al., Specific Binding of alpha-Macroglobulin to Complement-Type Repeat CR4 of the Low-Density Lipoprotein Receptor-Related Protein, (2000) Biochemistry 39:10627-10633; Vash et al., Three Complement-Type Repeats of the Low-Density Lipoprotein Receptor-Related Protein Define a.Common Binding Site for RAP, PAI-1, and Lactoferrin, (1998) Blood 92(9):3277-3285; Dolmer et al., NMR Solution Structure of Complement-like Repeat CR3 from the Low Density Lipoprotein Receptor-related Protein, (2000) Journal of Biological Chemistry 275(5):3264-3269; Huang et al., NMR Solution Structure of Complement-like Repeat CR8 from the Low Density Lipoprotein Receptor-related Protein, (1999) Journal of Biological Chemistry 274(20):14130-14136; and Liu et al., Uptake of HIV-1 Tat protein mediated by low-density lipoprotein receptor-related protein disrupts the neuronal metabolic balance of the receptor ligands, (2000) Nature Medicine 6(12):1380-1387.

Other references regarding monomer domains also include the following publications and references cited therein: FitzGerald et al, Pseudomonas Exotoxin-mediated Selection Yields Cells with Altered Expression of Low Density Lipoprotein Receptor-related Protein, (1995) Journal of Cell Biology, 129: 1533-41; Willnow and Herz, Genetic deficiency in low density lipoprotein receptor-related protein confers cellular resistance to Pseudomonas exotoxin A, (1994) Journal of Cell Science, 107:719-726; Trommsdorf et al., Interaction of Cytosolic Adaptor Proteins with Neuronal Apolipoprotein E Receptors and the Amyloid Precursor Protein, (1998) Journal of Biological Chemistry, 273(5): 33556-33560; Stockinger et al.., The Low Density Lipoprotein Receptor Gene Family, (1998) Journal of Biological Chemistry, 273(48): 32213-32221; Obermoeller et al., Ca+2 and Receptor-associated Protein are independently required for proper folding and disulfide bond formation of the low density lipoprotein receptor-related protein, (1998) Journal of Biological Chemistry, 273(35):22374-22381; Sato et al., 39-kDa receptor-associated protein (RAP) facilitates secretion and ligand binding of extracellular region of very-low-density-lipoprotein receptor: implications for a distinct pathway from low-density-lipoprotein receptor, (1999) Biochem. J., 341:377-383; Avromoglu et al, Functional Expression of the Chicken Low Density Lipoprotein Receptor-related Protein in a mutant Chinese Hamster Ovary Cell Line Restores Toxicity of Pseudomonas Exotoxin A and Degradation of alpha2 Macroglobulin, (1998) Journal of Biological Chemistry, 273(11) 6057-6065; Kingsley and Krieger, Receptor-mediated endocytosis of low density lipoprotein: Somatic cell mutants define multiple genes required for expression of surface-receptor activity, (1984) PNAS USA, 81:5454-5458; Li et al, Differential Functions of Members of the Low Density Lipoprotein Receptor Family Suggests by their distinct endocystosis rates, (2001) Journal of Biological Chemistry 276(21):18000-18006; and, Springer, An Extracellular beta-Propeller Module Predicted in Lipoprotein and Scavenger Receptors, Tyrosine Kinases, Epidermal Growth Factor Precursor, and Extracellular Matrix Components, (1998) J. Mol. Biol. 283:837-862.

Polynucleotides (also referred to as nucleic acids) encoding the monomer domains are typically employed to make monomer domains via expression. Nucleic acids that encode monomer domains can be derived from a variety of different sources. Libraries of monomer domains can be prepared by expressing a plurality of different nucleic acids encoding naturally occurring monomer domains, altered monomer domains (i.e., monomer domain variants), or a combinations thereof.

The invention provides methods of identifying monomer domains that bind to a selected or desired ligand or mixture of ligands. In some embodiments, monomer domains and/or immuno-domains are identified or selected for a desired property (e.g., binding affinity) and then the monomer domains and/or immuno-domains are formed into multimers. *See, e.g.,* Figure 5. For those embodiments, any method resulting in selection of domains with a desired property (e.g., a specific binding property) can be used. For example, the methods can comprise providing a plurality of different nucleic acids, each nucleic acid encoding a monomer domain; translating the plurality of different nucleic acids, thereby providing a plurality of different monomer domains; screening the plurality of different monomer domains for binding of the desired ligand or a mixture of ligands; and, identifying members of the plurality of different monomer domains that bind the desired ligand or mixture of ligands.

As mentioned above, monomer domains can be naturally-occurring or altered (non-natural variants). The term "naturally occurring" is used herein to indicate that an object can be found in nature. For example, natural monomer domains can include human monomer domains or optionally, domains derived from different species or sources, e.g., mammals, primates, rodents, fish, birds, reptiles, plants, etc. The natural occurring monomer domains can be obtained by a number of methods, e.g., by PCR amplification of genomic DNA or cDNA.

Monomer domains of the present invention can be naturally-occurring domains or non-naturally occurring variants. Libraries of monomer domains employed in the practice of the present invention may contain naturally-occurring monomer domain, non-naturally occurring monomer domain variants, or a combination thereof.

Monomer domain variants can include ancestral domains, chimeric domains, randomized domains, mutated domains, and the like. For example, ancestral domains can be based on phylogenetic analysis. Chimeric domains are domains in which one or more regions are replaced by corresponding regions from other domains of the same family. Randomized domains are domains in which one or more regions are randomized. The randomization can be based on full randomization, or optionally, partial randomization based on natural distribution.

The non-natural monomer domains or altered monomer domains can be produced by a number of methods. Any method of mutagenesis, such as site-directed mutagenesis and random mutatgenesis (e.g., chemical mutagenesis) can be used to produce variants. In some embodiments, error-prone PCR is employed to create variants. Additional methods include aligning a plurality of naturally occurring monomer domains by aligning conserved amino acids in the plurality of naturally occurring monomer domains; and, designing the non-naturally occurring monomer domain by maintaining the conserved amino acids and inserting, deleting or altering amino acids around the conserved amino acids to generate the non-naturally occurring monomer domain. In one embodiment, the conserved amino acids comprise cysteines. In another embodiment, the inserting step uses random amino acids, or optionally, the inserting step uses portions of the naturally occurring monomer domains. Amino acids can be inserted synthetically or can be encoded by a nucleic acid.

Nucleic acids encoding fragments of naturally-occurring monomer domains and/or immuno-domains can also be mixed and/or recombined (e.g., by using chemically or enzymatically-produced fragments) to generate full-length, modified monomer domains and/or immuno-domains. The fragments and the monomer domain can also be recombined by manipulating nucleic acids encoding domains or fragments thereof. For example, ligating a nucleic acid construct encoding fragments of the monomer domain can be used to generate an altered monomer domain.

Altered monomer domains can also be generated by providing a collection of synthetic oligonucleotides (e.g., overlapping oligonucleotides) encoding conserved, random, pseudorandom, or a defined sequence of peptide sequences that are then inserted by ligation into a predetermined site in a polynucleotide encoding a monomer domain. Similarly, the sequence diversity of one or more monomer domains can be expanded by mutating the monomer domain(s) with site-directed mutagenesis, random mutation, pseudorandom mutation, defined kernal mutation, codon-based mutation, and the like. The resultant nucleic acid molecules can be propagated in a host for cloning and amplification. The nucleic acids may be shuffled.

Further described herein is a method for recombining a plurality of nucleic acids encoding monomer domains and screening the resulting library for monomer domains that bind to the desired ligand or mixture of ligands or the like. Selected monomer domain nucleic acids can also be back-crossed by shuffling with polynucleotide sequences encoding neutral sequences (i.e., having insubstantial functional effect on binding), such as for example, by back-crossing with a wild-type or naturally-occurring sequence substantially identical to a selected sequence to produce native-like functional monomer domains. Generally, during back-crossing, subsequent selection is applied to retain the property, e.g., binding to the ligand.

As described herein the monomer library may be prepared by shuffling. In such a case, monomer domains are isolated and shuffled to combinatorially recombine the nucleic acid sequences that encode the monomer domains (recombination can occur between or within monomer domains, or both). The first step involves identifying a monomer domain having the desired property, e.g., affinity for a certain ligand. While maintaining the conserved amino acids during the recombination, the nucleic acid sequences encoding the monomer domains can be recombined, or recombined and joined into multimers.

Selection of monomer domains and/or immuno-domains from a library of domains can be accomplished by a variety of procedures. For example, one method of identifying monomer domains and/or immuno-domains which have a desired property involves translating a plurality of nucleic acids, where each nucleic acid encodes a monomer domain and/or immuno-domain, screening the polypeptides encoded by the plurality of nucleic acids, and identifying those monomer domains and/or immuno-domains that, e.g., bind to a desired ligand or mixture of ligands, thereby producing a selected monomer domain and/or immuno-domain. The monomer domains and/or immuno-domains expressed by each of the nucleic acids can be tested for their ability to bind to the ligand by methods known in the art (i.e. panning, affinity chromatography, FACS analysis).

As mentioned above, selection of monomer domains and/or immuno-domains can be based on binding to a ligand such as a target protein or other target molecule (e.g., lipid, carbohydrate, nucleic acid and the like). Other molecules can optionally be included in the methods along with the target, e.g., ions such as Ca⁺². The ligand can be a known ligand, e.g., a ligand known to bind one of the plurality of monomer domains, or e.g., the desired ligand can be an unknown monomer domain ligand. *See, e*.*g*., Figure 4, which illustrates some of the ligands that bind to the A-domain. Other selections of monomer domains and/or immuno-domains can be based, e.g., on inhibiting or enhancing a specific function of a target protein or an activity. Target protein activity can include, e.g., endocytosis or internalization, induction of second messenger system, up-regulation or down-regulation of a gene, binding to an extracellular matrix, release of a molecule(s), or a change in conformation. In this case, the ligand does not need to be known. The selection can also include using high-throughput assays.

When a monomer domain and/or immuno-domain is selected based on its ability to bind to a ligand, the selection basis can include selection based on a slow dissociation rate, which is usually predictive of high affinity. The valency of the ligand can also be varied to control the average binding affinity of selected monomer domains and/or immuno-domains. The ligand can be bound to a surface or substrate at varying densities, such as by including a competitor compound, by dilution, or by other method known to those in the art. High density (valency) of predetermined ligand can be used to enrich for monomer domains that have relatively low affinity, whereas a low density (valency) can preferentially enrich for higher affinity monomer domains.

A variety of reporting display vectors or systems can be used to express nucleic acids encoding the monomer domains immuno-domains and/or multimers of the present invention and to test for a desired activity. For example, a phage display system is a system in which monomer domains are expressed as fusion proteins on the phage surface (Pharmacia, Milwaukee Wis.). Phage display can involve the presentation of a polypeptide sequence encoding monomer domains and/or immuno-domains on the surface of a filamentous bacteriophage, typically as a fusion with a bacteriophage coat protein.

Generally in these methods, each phage particle or cell serves as an individual library member displaying a single species of displayed polypeptide in addition to the natural phage or cell protein sequences. The plurality of nucleic acids are cloned into the phage DNA at a site which results in the transcription of a fusion protein, a portion of which is encoded by the plurality of the nucleic acids. The phage containing a nucleic acid molecule undergoes replication and transcription in the cell. The leader sequence of the fusion protein directs the transport of the fusion protein to the tip of the phage particle. Thus, the fusion protein that is partially encoded by the nucleic acid is displayed on the phage particle for detection and selection by the methods described above and below. For example, the phage library can be incubated with a predetermined (desired) ligand, so that phage particles which present a fusion protein sequence that binds to the ligand can be differentially partitioned from those that do not present polypeptide sequences that bind to the predetermined ligand. For example, the separation can be provided by immobilizing the predetermined ligand. The phage particles (i.e., library members) which are bound to the immobilized ligand are then recovered and replicated to amplify the selected phage subpopulation for a subsequent round of affinity enrichment and phage replication. After several rounds of affinity enrichment and phage replication, the phage library members that are thus selected are isolated and the nucleotide sequence encoding the displayed polypeptide sequence is determined, thereby identifying the sequence(s) of polypeptides that bind to the predetermined ligand. Such methods are further described in PCT patent publication Nos. 91/17271, 91/18980, and 91/19818 and 93/08278.

Examples of other display systems include ribosome displays, a nucleotide-linked display (*see, e.g.,* U.S. Patent Nos. 6,281,344; 6,194,550, 6,207,446, 6,214,553, and 6,258,558), cell surface displays and the like. The cell surface displays include a variety of cells, e.g., E. coli, yeast and/or mammalian cells. When a cell is used as a display, the nucleic acids, e.g., obtained by PCR amplification followed by digestion, are introduced into the cell and translated. Optionally, polypeptides encoding the monomer domains or the multimers of the present invention can be introduced, e.g., by injection, into the cell.

The invention also includes compositions that are produced by methods of the the present invention. For example, the present invention includes monomer domains selected or identified from a library and/or libraries comprising monomer domains produced by the methods of the present invention.

The present invention also provides libraries of monomer domains. The libraries can include, e.g., about 100, 250, 500 monomer domains, or the library can include, e.g., about 100, 250, 500 or more polypeptides that encode monomer domains. Libraries can include monomer domains containing the same cysteine frame, e.g., A-domains.

As described herein, the libraries can be screened for a desired property such as binding of a desired ligand or mixture of ligands. For example, members of the library of monomer domains can be displayed and prescreened for binding to a known or unknown ligand or a mixture of ligands. The monomer domain sequences can then be mutagenized(e.g., recombined, chemically altered, etc.) or otherwise altered and the new monomer domains can be screened again for binding to the ligand or the mixture of ligands with an improved affinity. The selected monomer domains can be combined or joined to form multimers, which can then be screened for an improved affinity or avidity or altered specificity for the ligand or the mixture of ligands. Altered specificity can mean that the specificity is broadened, e.g., binding of multiple related viruses, or optionally, altered specificity can mean that the specificity is narrowed, e.g., binding within a specific region of a ligand. Those of skill in the art will recognize that there are a number of methods available to calculate avidity. *See, e.g.,* Mammen et al., Angew Chem Int. Ed. 37:2754-2794 (1998); Muller et al., Anal. Biochem. 261:149-158 (1998).

Those of skill in the art will recognize that the steps of generating variation and screening for a desired property can be repeated (i.e., performed recursively) to optimize results. For example, in a phage display library or other like format, a first screening of a library can be performed at relatively lower stringency, thereby selected as many particles associated with a target molecule as possible. The selected particles can then be isolated and the polynucleotides encoding the monomer or multimer can be isolated from the particles. Additional variations can then be generated from these sequences and subsequently screened at higher affinity. Figure 7 illustrates a generic cycle of selection and generation of variation.

Compositions polypeptides are included in the present invention. For example, the present invention provides a plurality of different nucleic acids wherein each nucleic acid encodes at least two LDL receptor class A monomer domains.

### 2. Multimers (also called Recombinant Mosaic Proteins or Combinatorial Mosaic Proteins)

Methods for generating multimers are a feature of the present invention. Multimers comprise at least two LDL receptor class A monomer domains. For example, multimers of the invention can comprise from 2 to about 10 monomer domains and/or immuno-domains, from 2 and about 8 monomer domains and/or immuno-domains, from about 3 and about 10 monomer domains and/or immuno-domains, about 7 monomer domains and/or immuno-domains, about 6 monomer domains and/or immuno-domains, about 5 monomer domains and/or immuno-domains, or about 4 monomer domains and/or immuno-domains. In some embodiments, the multimer comprises at least 3 monomer domains and/or immuno-domains. Typically, the monomer domains have been pre-selected for binding to the target molecule of interest.

In some embodiments, each monomer domain specifically binds to one target molecule. In some of these embodiments, each monomer binds to a different position (analogous to an epitope) on a target molecule. Multiple monomer domains and/or immuno-domains that bind to the same target molecule results in an avidity effect resulting in improved avidity of the multimer for the target molecule compared to each individual monomer. In some embodiments, the multimer has an avidity of at least about 1.5, 2, 3, 4, 5, 10, 20, 50 or 100 times the avidity of a monomer domain alone.

In another embodiment, the multimer comprises monomer domains with specificities for different target molecules. For example, multimers of such diverse monomer domains can specifically bind different components of a viral replication system or different serotypes of a virus. In some embodiments, at least one monomer domain binds to a toxin and at least one monomer domain binds to a cell surface molecule, thereby acting as a mechanism to target the toxin. In some embodiments, at least two monomer domains and/or immuno-domains of the multimer bind to different target molecules in a target cell or tissue. Similarly, therapeutic molecules can be targeted to the cell or tissue by binding a therapeutic agent to a monomer of the multimer that also contains other monomer domains and/or immuno-domains having cell or tissue binding specificity.

Multimers can comprise a variety of combinations of monomer domains. For example, in a single multimer, the selected monomer domains can be the same or identical, optionally, different or non-identical. In addition, the selected monomer domains can comprise various different monomer domains from the same monomer domain family, or various monomer domains from different domain families, or optionally, a combination of both.

Multimers that are generated in the practice of the present invention may be any of the following:
(1) A homo-multimer (a multimer of the same domain, i.e., A1-A1-A1-A1);
(2) A hetero-multimer of different domains of the same domain class, e.g., A1-A2-A3-A4. For example, hetero-multimer include multimers where A1, A2, A3 and A4 are different non-naturally occurring variants of a particular LDL-receptor class A domains, or where some of A1, A2, A3, and A4 are naturally-occurring variants of a LDL-receptor class A domain (*see, e.g.,* Figure 10).

Multimer libraries employed in the practice of the present invention may contain homo-multimers, hetero-multimers of different monomer domains (natural or non-natural) of LDL receptor class A domains.

Domains need not be selected before the domains are linked to form multimers. On the other hand, the domains can be selected for the ability to bind to a target molecule before before linked into multimers. Thus, for example, a multimer can comprise two domains that bind to one target molecule and a third domain that binds to a second target molecule.

The selected monomer domains are joined by a linker to form a multimer. For example, a linker is positioned between each separate discrete monomer domain in a multimer.

Joining the selected monomer domains via a linker can be accomplished using a variety of techniques known in the art. For example, combinatorial assembly of polynucleotides encoding selected monomer domains can be achieved by DNA ligation, or optionally, by PCR-based, self-priming overlap reactions. The linker can be attached to a monomer before the monomer is identified for its ability to bind to a target multimer or after the monomer has been selected for the ability to bind to a target multimer.

The linker can be naturally-occurring, synthetic or a combination of both. For example, the synthetic linker can be a randomized linker, e.g., both in sequence and size. In one aspect, the randomized linker can comprise a fully randomized sequence, or optionally, the randomized linker can be based on natural linker sequences. The linker can comprise, e.g,. a non-polypeptide moiety, a polynucleotide, a polypeptide or the like.

A linker can be rigid, or alternatively, flexible, or a combination of both. Linker flexibility can be a function of the composition of both the linker and the monomer domains that the linker interacts with. The linker joins two selected monomer domain, and maintains the monomer domains as separate discrete monomer domains. The linker can allow the separate discrete monomer domains to cooperate yet maintain separate properties such as multiple separate binding sites for the same ligand in a multimer, or e.g., multiple separate binding sites for different ligands in a multimer.

Choosing a suitable linker for a specific case where two or more monomer domains (i.e. polypeptide chains) are to be connected may depend on a variety of parameters including, e.g. the nature of the monomer domains, the structure and nature of the target to which the polypeptide multimer should bind and/or the stability of the peptide linker towards proteolysis and oxidation.

The present invention provides methods for optimizing the choice of linker once the desired monomer domains/variants have been identified. Generally, libraries of multimers having a composition that is fixed with regard to monomer domain composition, but variable in linker composition and length, can be readily prepared and screened as described above.

Typically, the linker polypeptide may predominantly include amino acid residues selected from the group consisting of Gly, Ser, Ala and Thr. For example, the peptide linker may contain at least 75% (calculated on the basis of the total number of residues present in the peptide linker), such as at least 80%, e.g. at least 85% or at least 90% of amino acid residues selected from the group consisting of Gly, Ser, Ala and Thr. The peptide linker may also consist of Gly, Ser, Ala and/or Thr residues only. The linker polypeptide should have a length, which is adequate to link two monomer domains in such a way that they assume the correct conformation relative to one another so that they retain the desired activity, for example as antagonists of a given receptor.

A suitable length for this purpose is a length of at least one and typically fewer than about 50 amino acid residues, such as 2-25 amino acid residues, 5-20 amino acid residues, 5-15 amino acid residues, 8-12 amino acid residues or 11 residues. Similarly, the polypeptide encoding a linker can range in size, e.g., from about 2 to about 15 amino acids, from about 3 to about 15, from about 4 to about 12, about 10, about 8, or about 6 amino acids. In methods and compositions involving nucleic acids, such as DNA, RNA, or combinations of both, the polynucleotide containing the linker sequence can be, e.g., between about 6 nucleotides and about 45 nucleotides, between about 9 nucleotides and about 45 nucleotides, between about 12 nucleotides and about 36 nucleotides, about 30 nucleotides, about 24 nucleotides, or about 18 nucleotides. Likewise, the amino acid residues selected for inclusion in the linker polypeptide should exhibit properties that do not interfere significantly with the activity or function of the polypeptide multimer. Thus, the peptide linker should on the whole not exhibit a charge which would be inconsistent with the activity or function of the polypeptide multimer, or interfere with internal folding, or form bonds or other interactions with amino acid residues in one or more of the monomer domains which would seriously impede the binding of the polypeptide multimer to the target in question.

As described herein, the peptide linker is selected from a library where the amino acid residues in the peptide linker are randomized for a specific set of monomer domains in a particular polypeptide multimer. A flexible linker could be used to find suitable combinations of monomer domains, which is then optimized using this random library of variable linkers to obtain linkers with optimal length and geometry. The optimal linkers may contain the minimal number of amino acid residues of the right type that participate in the binding to the target and restrict the movement of the monomer domains relative to each other in the polypeptide multimer when not bound to the target.

The use of naturally occurring as well as artificial peptide linkers to connect polypeptides into novel linked fusion polypeptides is well known in the literature (Hallewell et al. (1989), J. Biol. Chem. 264, 5260-5268; Alfthan et al. (1995), Protein Eng. 8, 725-731; Robinson & Sauer (1996), Biochemistry 35, 109-116; Khandekar et al. (1997), J. Biol. Chem. 272, 32190-32197; Fares et al. (1998), Endocrinology 139,2459-2464; Smallshaw et al. (1999), Protein Eng. 12, 623-630; US 5,856,456).

One example where the use of peptide linkers is widespread is for production of single-chain antibodies where the variable regions of a light chain (V_{L}) and a heavy chain (V_{H}) are joined through an artificial linker, and a large number of publications exist within this particular field. A widely used peptide linker is a 15mer consisting of three repeats of a Gly-Gly-Gly-Gly-Ser amino acid sequence ((Gly₄Ser)₃). Other linkers have been used and phage display technology as well as selective infective phage technology has been used to diversify and select appropriate linker sequences (Tang et al. (1996), J. Biol. Chem. 271, 15682-15686; Hennecke et al. (1998), Protein Eng. 11, 405-410). Peptide linkers have been used to connect individual chains in hetero- and homo-dimeric proteins such as the T-cell receptor, the lambda Cro repressor, the P22 phage Arc repressor, IL-12, TSH, FSH, IL-5, and interferon-γ. Peptide linkers have also been used to create fusion polypeptides. Various linkers have been used and in the case of the Arc repressor phage display has been used to optimize the linker length and composition for increased stability of the single-chain protein (Robinson and Sauer (1998), Proc. Natl. Acad. Sci. USA 95, 5929-5934).

Another type of linker is an intein, i.e. a peptide stretch which is expressed with the single-chain polypeptide, but removed post-translationally by protein splicing. The use of inteins is reviewed by F.S. Gimble in Chemistry and Biology, 1998, Vol 5, No. 10 pp. 251-256.

Still another way of obtaining a suitable linker is by optimizing a simple linker, e.g. (Gly₄Ser)ₙ, through random mutagenesis.

As mentioned above, it is generally preferred that the peptide linker possess at least some flexibility. Accordingly, in some embodiments, the peptide linker contains 1-25 glycine residues, 5-20 glycine residues, 5-15 glycine residues or 8-12 glycine residues. The peptide linker will typically contain at least 50% glycine residues, such as at least 75% glycine residues. The peptide linker may comprise glycine residues only.

The peptide linker may, in addition to the glycine residues, comprise other residues, in particular residues selected from the group consisting of Ser, Ala and Thr, in particular Ser. Thus, one example of a specific peptide linker includes a peptide linker having the amino acid sequence Glyₓ-Xaa-Gly_{y}-Xaa-Gly_{z}, wherein each Xaa is independently selected from the group consisting Ala, Val, Leu, Ile, Met, Phe, Trp, Pro, Gly, Ser, Thr, Cys, Tyr, Asn, Gln, Lys, Arg, His, Asp and Glu, and wherein x, y and z are each integers in the range from 1-5. In some embodiments, each Xaa is independently selected from the group consisting of Ser, Ala and Thr, in particular Ser. More particularly, the peptide linker has the amino acid sequence Gly-Gly-Gly-Xaa-Gly-Gly-Gly-Xaa-Gly-Gly-Gly, wherein each Xaa is independently selected from the group consisting Ala, Val, Leu, Ile, Met, Phe, Trp, Pro, Gly, Ser, Thr, Cys, Tyr, Asn, Gln, Lys, Arg, His, Asp and Glu. As described herein, each Xaa can be independently selected from the group consisting of Ser, Ala and Thr, in particular Ser.

In some cases it may be desirable or necessary to provide some rigidity into the peptide linker. This may be accomplished by including proline residues in the amino acid sequence of the peptide linker. Thus, the peptide linker may comprise at least one proline residue in the amino acid sequence of the peptide linker. For example, the peptide linker has an amino acid sequence, wherein at least 25%, such as at least 50%, e.g. at least 75%, of the amino acid residues are proline residues. As described herein, the peptide linker comprises proline residues only.

Also described herein are peptide linkers modified in such a way that an amino acid residue comprising an attachment group for a non-polypeptide moiety is introduced. Examples of such amino acid residues may be a cysteine residue (to which the non-polypeptide moiety is then subsequently attached) or the amino acid sequence may include an *in vivo* N-glycosylation site (thereby attaching a sugar moiety (*in vivo*) to the peptide linker).

The peptide linker may comprise at least one cysteine residue, such as one cysteine residue. Thus, in some embodiments of the invention the peptide linker comprises amino acid residues selected from the group consisting of Gly, Ser, Ala, Thr and Cys. Such a peptide linker may comprise one cysteine residue only.

The peptide linker described herein may comprise glycine residues and cysteine residue, such as glycine residues and cysteine residues only. Typically, only one cysteine residue will be included per peptide linker. Thus, one example of a specific peptide linker comprising a cysteine residue, includes a peptide linker having the amino acid sequence Glyₙ-Cys-Glyₘ, wherein n and m are each integers from 1-12, e.g., from 3-9, from 4-8, or from 4-7. More particularly, the peptide linker may have the amino acid sequence GGGGG-C-GGGGG.

This approach (i.e. introduction of an amino acid residue comprising an attachment group for a non-polypeptide moiety) may also be used for the more rigid proline-containing linkers. Accordingly, the peptide linker may comprise proline and cysteine residues, such as proline and cysteine residues only. An example of a specific proline-containing peptide linker comprising a cysteine residue, includes a peptide linker having the amino acid sequence Proₙ-Cys-Proₘ, wherein n and m are each integers from 1-12, preferably from 3-9, such as from 4-8 or from 4-7. More particularly, the peptide linker may have the amino acid sequence PPPPP-C-PPPPP.

The purpose of introducing an amino acid residue, such as a cysteine residue, comprising an attachment group for a non-polypeptide moiety is to subsequently attach a non-polypeptide moiety to said residue. For example, non-polypeptide moieties can improve the serum half-life of the polypeptide multimer. Thus, the cysteine residue can be covalently attached to a non-polypeptide moiety. Preferred examples of non-polypeptide moieties include polymer molecules, such as PEG or mPEG, in particular mPEG as well as non-polypeptide therapeutic agents.

The skilled person will acknowledge that amino acid residues other than cysteine may be used for attaching a non-polypeptide to the peptide linker. One particular example of such other residue includes coupling the non-polypeptide moiety to a lysine residue.

Another possibility of introducing a site-specific attachment group for a non-polypeptide moiety in the peptide linker is to introduce an *in vivo* N-glycosylation site, such as one *in vivo* N-glycosylation site, in the peptide linker. For example, an *in vivo* N-glycosylation site may be introduced in a peptide linker comprising amino acid residues selected from the group consisting of Gly, Ser, Ala and Thr. It will be understood that in order to ensure that a sugar moiety is in fact attached to said *in vivo* N-glycosylation site, the nucleotide sequence encoding the polypeptide multimer must be inserted in a glycosylating, eukaryotic expression host.

A specific example of a peptide linker comprising an *in vivo* N-glycosylation site is a peptide linker having the amino acid sequence Glyₙ-Asn-Xaa-Ser/Thr-Glyₘ, preferably Glyₙ-Asn-Xaa-Thr-Glyₘ, wherein Xaa is any amino acid residue except proline, and wherein n and m are each integers in the range from 1-8, preferably in the range from 2-5.

Often, the amino acid sequences of all peptide linkers present in the polypeptide multimer will be identical. Nevertheless, in certain linkers the amino acid sequences of all peptide linkers present in the polypeptide multimer may be different. The latter is believed to be particular relevant in case the polypeptide multimer is a polypeptide tri-mer or tetra-mer and particularly in such cases where an amino acid residue comprising an attachment group for a non-polypeptide moiety is included in the peptide linker.

Quite often, it will be desirable or necessary to attach only a few, typically only one, non-polypeptide moieties/moiety (such as mPEG, a sugar moiety or a non-polypeptide therapeutic agent) to the polypeptide multimer in order to achieve the desired effect, such as prolonged serum-half life. Evidently, in case of a polypeptide tri-mer, which will contain two peptide linkers, only one peptide linker is typically required to be modified, e.g. by introduction of a cysteine residue, whereas modification of the other peptide linker will typically not be necessary not. In this case all (both) peptide linkers of the polypeptide multimer (tri-mer) are different.

Accordingly, as described herein, the amino acid sequences of all peptide linkers present in the polypeptide multimer are identical except for one, two or three peptide linkers, such as except for one or two peptide linkers, in particular except for one peptide linker, which has/have an amino acid sequence comprising an amino acid residue comprising an attachment group for a non-polypeptide moiety. Preferred examples of such amino acid residues include cysteine residues of *in vivo* N-glycosylation sites.

A linker can be a native or synthetic linker sequence. An exemplary native linker includes, e.g., the sequence between the last cysteine of a first LDL receptor A domain and the first cysteine of a second LDL receptor A domain can be used as a linker sequence. Analysis of various A domain linkages reveals that native linkers range from at least 3 amino acids to fewer than 20 amino acids, e.g., 4, 5, 6, 7, 8, 9, 10, 11, 12, 13, 14, 15, 16,17, or 18 amino acids long. However, those of skill in the art will recognize that longer or shorter linker sequences can be used. An exemplary A domain linker sequence is depicted in Figure 8. The linker is a 6-mer of the following sequence A₁A₂A₃A₄A₅A₆, wherein A₁ is selected from the amino acids A, P, T, Q, E and K; A₂ and A₃ are any amino acid except C, F, Y, W, or M; A₄ is selected from the amino acids S, G and R; A₅ is selected from the amino acids H, P, and R; and A₆ is the amino acid, T.

Methods for generating multimers from monomer domains can include joining the selected domains with at least one linker to generate at least one multimer, e.g., the multimer can comprise at least two of the monomer domains and the linker. The multimer(s) is then screened for an improved avidity or affinity or altered specificity for the desired ligand or mixture of ligands as compared to the selected monomer domains.

In other methods described herein, the selected multimer domains are joined with at least one linker to generate at least two multimers, wherein the two multimers comprise two or more of the selected monomer domains and the linker. The two or more multimers are screened for an improved avidity or affinity or altered specificity for the desired ligand or mixture of ligands as compared to the selected monomer domains, Compositions of two or more multimers produced by the above method are also described herein.

Typically, multimers of the present invention are a single discrete polypeptide. Multimers of partial linker-domain-partial linker moieties are an association of multiple polypeptides, each corresponding to a partial linker-domain-partial linker moiety.

In some polypeptides described herein, the selected multimer comprises more than two domains. Such multimers can be generated in a step fashion, e.g., where the addition of each new domain is tested individually and the effect of the domains is tested in a sequential fashion. *See*, *e*.*g*., Figure 6. In other polypeptides described herein, domains are linked to form multimers comprising more than two domains and selected for binding without prior knowledge of how smaller multimers, or alternatively, how each domain, bind.

The methods described herein also include methods of evolving multimers. The methods can comprise, e.g., any or all of the following steps: providing a plurality of different nucleic acids, where each nucleic acid encoding a monomer domain; translating the plurality of different nucleic acids, which provides a plurality of different monomer domains; screening the plurality of different monomer domains for binding of the desired ligand or mixture of ligands; identifying members of the plurality of different monomer domains that bind the desired ligand or mixture of ligands, which provides selected monomer domains; joining the selected monomer domains with at least one linker to generate at least one multimer, wherein the at least one multimer comprises at least two of the selected monomer domains and the at least one linker; and, screening the at least one multimer for an improved affinity or avidity or altered specificity for the desired ligand or mixture of ligands as compared to the selected monomer domains.

Additional variation can be introduced by inserting linkers of different length and composition between domains. This allows for the selection of optimal linkers between domains. In some methods described herein optimal length and composition of linkers will allow for optimal binding of domains. In some methods described herein the domains with a particular binding affinity(s) are linked via different linkers and optimal linkers are selected in a binding assay. For example, domains are selected for desired binding properties and them formed into a library comprising a variety of linkers. The library can then be screened to identify opitmal linkers. Alternatively, multimer libraries can be formed where the effect of domain or linker on target molecule binding is not known.

Methods described herein also include generating one or more selected multimers by providing a plurality of monomer domains. The plurality of monomer domains are screened for binding of a desired ligand or mixture of ligands. Members of the plurality of domains that bind the desired ligand or mixture of ligands are identified, thereby providing domains with a desired affinity. The identified domains are joined with at least one linker to generate the multimers, wherein each multimer comprises at least two of the selected domains and the at least one linker; and, the multimers are screened for an improved affinity or avidity or altered specificity for the desired ligand or mixture of ligands as compared to the selected domains, thereby identifying the one or more selected multimers.

Selection of multimers can be accomplished using a variety of techniques including those mentioned above for identifying monomer domains. Other selection methods include, e.g., a selection based on an improved affinity or avidity or altered specificity for the ligand compared to selected monomer domains. For example, a selection can be based on selective binding to specific cell types, or to a set of related cells or protein types (e.g., different virus serotypes). Optimization of the property selected for, e.g., avidity of a ligand, can then be achieved by recombining the domains, as well as manipulating amino acid sequence of the individual monomer domains or the linker domain or the nucleotide sequence encoding such domains, as mentioned in the present invention.

One method for identifying multimers can be accomplished by displaying the multimers. As with the monomer domains, the multimers are optionally expressed or displayed on a variety of display systems, e.g., phage display, ribosome display, nucleotide-linked display (*see, e.g.,* U.S. Patent Nos. 6,281,344; 6,194,550, 6,207,446, 6,214,553, and 6,258,558) and/or cell surface display, as described above. Cell surface displays can include but are not limited to *E. coli,* yeast or mammalian cells. In addition, display libraries of multimers with multiple binding sites can be panned for avidity or affinity or altered specificity for a ligand or for multiple ligands.

Other variations include the use of multiple binding compounds, such that monomer domains, multimers or libraries of these molecules can be simultaneously screened for a multiplicity of ligands or compounds that have different binding specificity. Multiple predetermined ligands or compounds can be concomitantly screened in a single library, or sequential screening against a number of monomer domains or multimers. In one variation, multiple ligands or compounds, each encoded on a separate bead (or subset of beads), can be mixed and incubated with monomer domains, multimers or libraries of these molecules under suitable binding conditions. The collection of beads, comprising multiple ligands or compounds, can then be used to isolate, by affinity selection, selected monomer domains, selected multimers or library members. Generally, subsequent affinity screening rounds can include the same mixture of beads, subsets thereof, or beads containing only one or two individual ligands or compounds. This approach affords efficient screening, and is compatible with laboratory automation, batch processing, and high throughput screening methods.

As described herein, multimers can be simultaneously screened for the ability to bind multiple ligands, wherein each ligand comprises a different label. For example, each ligand can be labeled with a different fluorescent label, contacted simultaneously with a multimer or multimer library. Multimers with the desired affinity are then identified (e.g., by FACS sorting) based on the presence of the labels linked to the desired labels.

The selected multimers of the above methods can be further manipulated, e.g., by recombining or shuffling the selected multimers (recombination can occur between or within multimers or both), mutating the selected multimers, and the like. This results in altered multimers which then can be screened and selected for members that have an enhanced property compared to the selected multimer, thereby producing selected altered multimers.

Linkers, multimers or selected multimers produced by the methods indicated above and below are described herein. Libraries comprising multimers, e.g, a library comprising about 100, 250, 500 or more members produced by the methods of the present invention or selected by the methods of the present invention are provided. As described herein, one or more cell comprising members of the libraries, are also included. Libraries of the recombinant polypeptides are also, described herein e.g., a library comprising about 100, 250, 500 or more different recombinant polypetides.

Compositions described herein can be bound to a matrix of an affinity material, e.g., the recombinant polypeptides. Examples of affinity material include, e.g., beads, a column, a solid support, and/or the like.

Suitable linkers as described herein include an obligate heterodimer of partial linker moieties. The term "obligate heterodimer" refers herein to a dimer of two partial linker moieties that differ from each other in composition, and which associate with each other in a non-covalent, specific manner to join two domains together. The specific association is such that the two partial linkers associate substantially with each other as compared to associating with other partial linkers. Thus, in contrast to multimers of the present invention that are expressed as a single polypeptide, multimers of domains that are linked together via heterodimers are assembled from discrete partial linker-monomer-partial linker units. Assembly of the heterodimers can be achieved by, for example, mixing. Thus, if the partial linkers are polypeptide segments, each partial linker-monomer-partial linker unit may be expressed as a discrete peptide prior to multimer assembly. A disulfide bond can be added to covalently lock the peptides together following the correct non-covalent pairing. A multimer containing such obligate heterodimers is depicted in Figure 12. Partial linker moieties that are appropriate for forming obligate heterodimers include, for example, polynucleotides, polypeptides, and the like. For example, when the partial linker is a polypeptide, binding domains are produced individually along with their unique linking peptide (i.e., a partial linker) and later combined to form multimers. The spacial order of the binding domains in the multimer is thus mandated by the heterodimeric binding specificity of each partial linker. Partial linkers can contain terminal amino acid sequences that specifically bind to a defined heterologous amino acid sequence. An example of such an amino acid sequence is the Hydra neuropeptide head activator as described in Bodenmuller et al., The neuropeptide head activator loses its biological activity by dimerization, (1986) EMBO J 5(8):1825-1829. *See, e*.*g*., U.S. Patent No. 5,491,074 and WO 94/28173. These partial linkers allow the multimer to be produced first as monomer-partial linker units or partial linker-monomer-partial linker units that are then mixed together and allowed to assemble into the ideal order based on the binding specificities of each partial linker.

When the partial linker comprises a DNA binding motiff, each monomer domain has an upstream and a downstream partial linker (i.e., Lp-domain-Lp, where "Lp" is a representation of a partial linker) that contains a DNA binding protein with exclusively unique DNA binding specificity. These domains can be produced individually and then assembled into a specific multimer by the mixing of the domains with DNA fragments containing the proper nucleotide sequences (i.e., the specific recognition sites for the DNA binding proteins of the partial linkers of the two desired domains) so as to join the domains in the desired order. Additionally, the same domains may be assembled into many different multimers by the addition of DNA sequences containing various combinations of DNA binding protein recognition sites. Further randomization of the combinations of DNA binding protein recognition sites in the DNA fragments can allow the assembly of libraries of multimers. The DNA can be synthesized with backbone analogs to prevent degradation in vivo.

A significant advantage of the present invention is that known ligands, or unknown ligands can be used to select the monomer domains and/or multimers. No prior information regarding ligand structure is required to isolate the monomer domains of interest or the multimers of interest. The monomer domains, identified can have biological activity, which is meant to include at least specific binding affinity for a selected or desired ligand, and, in some instances, will further include the ability to block the binding of other compounds, to stimulate or inhibit metabolic pathways, to act as a signal or messenger, to stimulate or inhibit cellular activity, and the like.

A single ligand can be used, or optionally a variety of ligands can be used to select the monomer domains, and/or multimers. A monomer domain of the present invention can bind a single ligand or a variety of ligands. A multimer of the present invention can have multiple discrete binding sites for a single ligand, or optionally, can have multiple binding sites for a variety of ligands.

The potential applications of multimers of the present invention are diverse. For example, the invention can be used in the application for creating antagonists, where the selected monomer domains or multimers block the interaction between two proteins. Optionally, the invention can generate agonists. For example, multimers binding two different proteins, e.g., enzyme and substrate, can enhance protein function, including, for example, enzymatic activity and/or substrate conversion.

Other applications include cell targeting. For example, multimers consisting of monomer domains and/or immuno-domains that recognize specific cell surface proteins can bind selectively to certain cell types. Applications involving monomer domains and/or immuno-domains as antiviral agents are also included. For example, multimers binding to different epitopes on the virus particle can be useful as antiviral agents because of the polyvalency. Other applications can include, but are not limited to, protein purification, protein detection, biosensors, ligand-affinity capture experiments and the like. Furthermore, domains or multimers can be synthesized in bulk by conventional means for any suitable use, e.g., as a therapeutic or diagnostic agent.

As described herein, the multimer comprises monomer domains with specificities for different proteins. The different proteins can be related or unrelated. Examples of related proteins including members of a protein family or different serotypes of a virus. Alternatively, the monomer domains and/or immuno-domains of a multimer can target different molecules in a physiological pathway (e.g., different blood coagulation proteins). As further described herein, monomer domains and/or immuno-domains bind to proteins in unrelated pathways (e.g., two domains bind to blood factors, two other domains bind to inflammation-related proteins and a fifth binds to serum albumin).

### 3. Therapeutic and Prophylactic Treatment Method - Included for Reference Only

The present patent also describes methods of therapeutically or prophylactically treating a disease or disorder by administering *in vivo* or *ex vivo* one or more nucleic acids or polypeptides of the invention described above (or compositions comprising a pharmaceutically acceptable excipient and one or more such nucleic acids or polypeptides) to a subject, including, *e*.*g*., a mammal, including a human, primate, mouse, pig, cow, goat, rabbit, rat, guinea pig, hamster, horse, sheep; or a non-mammalian vertebrate such as a bird (*e*.*g*., a chicken or duck), fish, or invertebrate.

In *ex vivo* methods, one or more cells or a population of cells of interest of the subject (*e*.*g*., tumor cells, tumor tissue sample, organ cells, blood cells, cells of the skin, lung, heart, muscle, brain, mucosae, liver, intestine, spleen, stomach, lymphatic system, cervix, vagina, prostate, mouth, tongue, *etc*.) are obtained or removed from the subject and contacted with an amount of a selected monomer domain and/or multimer of the invention that is effective in prophylactically or therapeutically treating the disease, disorder, or other condition. The contacted cells are then returned or delivered to the subject to the site from which they were obtained or to another site (*e.g.*, including those defined above) of interest in the subject to be treated. If desired, the contacted cells can be grafted onto a tissue, organ, or system site (including all described above) of interest in the subject using standard and well-known grafting techniques or, *e*.*g*., delivered to the blood or lymph system using standard delivery or transfusion techniques.

Described herein are *in vivo* methods in which one or more cells or a population of cells of interest of the subject are contacted directly or indirectly with an amount of a selected monomer domain and/or multimer of the invention effective in prophylactically or therapeutically treating the disease, disorder, or other condition. In direct contact/administration formats, the selected monomer domain and/or multimer is typically administered or transferred directly to the cells to be treated or to the tissue site of interest (*e*.*g*., tumor cells, tumor tissue sample, organ cells, blood cells, cells of the skin, lung, heart, muscle, brain, mucosae, liver, intestine, spleen, stomach, lymphatic system, cervix, vagina, prostate; mouth, tongue, *etc*.) by any of a variety of formats, including topical administration, injection (*e*.*g*., by using a needle or syringe), or vaccine or gene gun delivery, pushing into a tissue, organ, or skin site. The selected monomer domain and/or multimer can be delivered, for example, intramuscularly, intradermally, subdermally, subcutaneously, orally, intraperitoneally, intrathecally, intravenously, or placed within a cavity of the body (including, *e*.*g*., during surgery), or by inhalation or vaginal or rectal administration.

In *in vivo* indirect contact/administration formats, the selected monomer domain and/or multimer is typically administered or transferred indirectly to the cells to be treated or to the tissue site of interest, including those described above (such as, *e*.*g.,* skin cells, organ systems, lymphatic system, or blood cell system, *etc*.), by contacting or administering the polypeptide of the invention directly to one or more cells or population of cells from which treatment can be facilitated. For example, tumor cells within the body of the subject can be treated by contacting cells of the blood or lymphatic system, skin, or an organ with a sufficient amount of the selected monomer domain and/or multimer such that delivery of the selected monomer domain and/or multimer to the site of interest (*e*.*g*., tissue, organ, or cells of interest or blood or lymphatic system within the body) occurs and effective prophylactic or therapeutic treatment results. Such contact, administration, or transfer is typically made by using one or more of the routes or modes of administration described above.

Further described herein are *ex vivo* methods in which one or more cells of interest or a population of cells of interest of the subject (*e*.*g*., tumor cells, tumor tissue sample, organ cells, blood cells, cells of the skin, lung, heart, muscle, brain, mucosae, liver, intestine, spleen, stomach, lymphatic system, cervix, vagina, prostate, mouth, tongue, *etc*.) are obtained or removed from the subject and transformed by contacting said one or more cells or population of cells with a polynucleotide construct comprising a nucleic acid sequence of the invention that encodes a biologically active polypeptide of interest (*e*.*g*., a selected monomer domain and/or multimer) that is effective in prophylactically or therapeutically treating the disease, disorder, or other condition. The one or more cells or population of cells is contacted with a sufficient amount of the polynucleotide construct and a promoter controlling expression of said nucleic acid sequence such that uptake of the polynucleotide construct (and promoter) into the cell(s) occurs and sufficient expression of the target nucleic acid sequence results to produce an amount of the biologically active polypeptide, encoding a selected monomer domain and/or multimer, effective to prophylactically or therapeutically treat the disease, disorder, or condition. The polynucleotide construct can include a promoter sequence (*e*.*g*., CMV promoter sequence) that controls expression of the nucleic acid sequence of the invention and/or, if desired, one or more additional nucleotide sequences encoding at least one or more of another polypeptide of the invention, a cytokine, adjuvant, or co-stimulatory molecule, or other polypeptide of interest.

Following transfection, the transformed cells are returned, delivered, or transferred to the subject to the tissue site or system from which they were obtained or to another site (*e*.*g*., tumor cells, tumor tissue sample, organ cells, blood cells, cells of the skin, lung, heart, muscle, brain, mucosae, liver, intestine, spleen, stomach, lymphatic system, cervix, vagina, prostate, mouth, tongue, *etc*.) to be treated in the subject. If desired, the cells can be grafted onto a tissue, skin, organ, or body system of interest in the subject using standard and well-known grafting techniques or delivered to the blood or lymphatic system using standard delivery or transfusion techniques. Such delivery, administration, or transfer of transformed cells is typically made by using one or more of the routes or modes of administration described above. Expression of the target nucleic acid occurs naturally or can be induced (as described in greater detail below) and an amount of the encoded polypeptide is expressed sufficient and effective to treat the disease or condition at the site or tissue system.

Also described herein are *in vivo* methods in which one or more cells of interest or a population of cells of the subject (*e*.*g*., including those cells and cells systems and subjects described above) are transformed in the body of the subject by contacting the cell(s) or population of cells with (or administering or transferring to the cell(s) or population of cells using one or more of the routes or modes of administration described above) a polynucleotide construct comprising a nucleic acid sequence of the invention that encodes a biologically active polypeptide of interest (*e*.*g*., a selected monomer domain and/or multimer) that is effective in prophylactically or therapeutically treating the disease, disorder, or other condition.

The polynucleotide construct can be directly administered or transferred to cell(s) suffering from the disease or disorder (*e*.*g*., by direct contact using one or more of the routes or modes of administration described above). Alternatively, the polynucleotide construct can be indirectly administered or transferred to cell(s) suffering from the disease or disorder by first directly contacting non-diseased cell(s) or other diseased cells using one or more of the routes or modes of administration described above with a sufficient amount of the polynucleotide construct comprising the nucleic acid sequence encoding the biologically active polypeptide, and a promoter controlling expression of the nucleic acid sequence, such that uptake of the polynucleotide construct (and promoter) into the cell(s) occurs and sufficient expression of the nucleic acid sequence of the invention results to produce an amount of the biologically active polypeptide effective to prophylactically or therapeutically treat the disease or disorder, and whereby the polynucleotide construct or the resulting expressed polypeptide is transferred naturally or automatically from the initial delivery site, system, tissue or organ of the subject's body to the diseased site, tissue, organ or system of the subject's body (*e*.*g*., via the blood or lymphatic system). Expression of the target nucleic acid occurs naturally or can be induced (as described in greater detail below) such that an amount of expressed polypeptide is sufficient and effective to treat the disease or condition at the site or tissue system. The polynucleotide construct can include a promoter sequence (*e*.*g*., CMV promoter sequence) that controls expression of the nucleic acid sequence and/or, if desired, one or more additional nucleotide sequences encoding at least one or more of another polypeptide of the invention, a cytokine, adjuvant, or co-stimulatory molecule, or other polypeptide of interest.

In each of the *in vivo* and *ex vivo* treatment methods as described above, a composition comprising an excipient and the polypeptide or nucleic acid of the invention can be administered or delivered. Also described herein, a composition comprising a pharmaceutically acceptable excipient and a polypeptide or nucleic acid can be administered or delivered to the subject as described above in an amount effective to treat the disease or disorder.

As further described herein, in each *in vivo* and *ex vivo* treatment method described above, the amount of polynucleotide administered to the cell(s) or subject can be an amount such that uptake of said polynucleotide into one or more cells of the subject occurs and sufficient expression of said nucleic acid sequence results to produce an amount of a biologically active polypeptide effective to enhance an immune response in the subject, including an immune response induced by an immunogen (*e*.*g*., antigen). For each such method, the amount of polypeptide administered to cell(s) or subject can be an amount sufficient to enhance an immune response in the subject, including that induced by an immunogen (*e*.*g*., antigen).

Further described herein are in vivo or in vivo treatment method in which a polynucleotide construct (or composition comprising a polynucleotide construct) is used to deliver a physiologically active polypeptide to a subject. The expression of the polynucleotide construct can be induced by using an inducible on- and off-gene expression system. Examples of such on- and off-gene expression systems include the Tet-On™ Gene Expression System and Tet-Off™ Gene Expression System (*see, e*.*g.,* Clontech Catalog 2000, pg. 110-111 for a detailed description of each such system), respectively. Other controllable or inducible on- and off-gene expression systems are known to those of ordinary skill in the art. With such system, expression of the target nucleic of the polynucleotide construct can be regulated in a precise, reversible, and quantitative manner. Gene expression of the target nucleic acid can be induced, for example, after the stable transfected cells containing the polynucleotide construct comprising the target nucleic acid are delivered or transferred to or made to contact the tissue site, organ or system of interest. Such systems are of particular benefit in treatment methods and formats in which it is advantageous to delay or precisely control expression of the target nucleic acid (*e*.*g*., to allow time for completion of surgery and/or healing following surgery; to allow time for the polynucleotide construct comprising the target nucleic acid to reach the site, cells, system, or tissue to be treated; to allow time for the graft containing cells transformed with the construct to become incorporated into the tissue or organ onto or into which it has been spliced or attached, *etc*.).

### 4. Further Manipulating Monomer Domains and/or Multimer Nucleic Acids and Polypeptides - Described herein for Reference Only

As mentioned above, the polypeptide of the present invention can be altered. Descriptions of a variety of diversity generating procedures for generating modified or altered nucleic acid sequences encoding these polypeptides are described above and below in the following publications and the references cited therein: Soong, N. et al., Molecular breeding of viruses, (2000) Nat Genet 25(4):436-439; Stemmer, et al., Molecular breeding of viruses for targeting and other clinical properties, (1999) Tumor Targeting 4:1-4; Ness et al., DNA Shuffling of subgenomic sequences of subtilisin, (1999) Nature Biotechnology 17:893-896; Chang et al, Evolution of a cytokine using DNA family shuffling, (1999) Nature Biotechnology 17:793-797; Minshull and Stemmer, Protein evolution by molecular breeding, (1999) Current Opinion in Chemical Biology 3:284-290; Christians et al., Directed evolution of thymidine kinase for AZT phosphorylation using DNA family shuffling, (1999) Nature Biotechnology 17:259-264; Crameri et al., DNA shuffling of a family of genes from diverse species accelerates directed evolution, (1998) Nature 391:288-291; Crameri et al., Molecular evolution of an arsenate detoxification pathway by DNA shuffling, (1997) Nature Biotechnology 15:436-438; Zhang et al., Directed evolution of an effective fucosidase from a galactosidase by DNA shuffling and screening (1997) Proc. Natl. Acad. Sci. USA 94:4504-4509; Patten et al., Applications of DNA Shuffling to Pharmaceuticals and Vaccines, (1997) Current Opinion in Biotechnology 8:724-733; Crameri et al, Construction and evolution of antibody-phage libraries by DNA shuffling, (1996) Nature Medicine 2:100-103; Crameri et al., Improved green fluorescent protein by molecular evolution using DNA shuffling, (1996) Nature Biotechnology 14:315-319; Gates et al., Affinity selective isolation of ligands from peptide libraries through display on a lac repressor 'headpiece dimer', (1996) Journal of Molecular Biology 255:373-386; Stemmer, Sexual PCR and Assembly PCR, (1996) In: The Encyclopedia of Molecular Biology. VCH Publishers, New York. pp.447-457; Crameri and Stemmer, Combinatorial multiple cassette mutagenesis creates all the permutations of mutant and wildtype cassettes, (1995) BioTechniques 18:194-195; Stemmer et al., Single-step assembly of a gene and entire plasmid form large numbers of oligodeoxy-ribonucleotides, (1995) Gene, 164:49-53; Stemmer, The Evolution of Molecular Computation, (1995) Science 270: 1510; Stemmer. Searching Sequence Space, (1995) Bio/Technology 13:549-553; Stemmer, Rapid evolution of a protein in vitro by DNA shuffling, (1994) Nature 370:389-391; and Stemmer, DNA shuffling by random fragmentation and reassembly: In vitro recombination for molecular evolution, (1994) Proc. Natl. Acad. Sci. USA 91:10747-10751.

Mutational methods of generating diversity include, for example, site-directed mutagenesis (Ling et al., Approaches to DNA mutagenesis: an overview, (1997) Anal Biochem. 254(2): 157-178; Dale et al., Oligonucleotide-directed random mutagenesis using the phosphorothioate method, (1996) Methods Mol. Biol. 57:369-374; Smith, In vitro mutagenesis, (1985) Ann. Rev. Genet. 19:423-462; Botstein & Shortle, Strategies and applications of in vitro mutagenesis, (1985) Science 229:1193-1201; Carter, Site-directed mutagenesis, (1986) Biochem. J. 237:1-7; and Kunkel, The efficiency of oligonucleotide directed mutagenesis, (1987) in Nucleic Acids & Molecular Biology (Eckstein, F. and Lilley, D.M.J. eds., Springer Verlag, Berlin)); mutagenesis using uracil containing templates (Kunkel, Rapid and efficient site-specific mutagenesis without phenotypic selection, (1985) Proc. Natl. Acad. Sci. USA 82:488-492; Kunkel et al., Rapid and efficient site-specific mutagenesis without phenotypic selection, (1987) Methods in Enzymol. 154, 367-382; and Bass et al., Mutant Trp repressors with new DNA-binding specificities, (1988) Science 242:240-245); oligonucleotide-directed mutagenesis ((1983) Methods in Enzymol. 100: 468-500; (1987) Methods in Enzymol. 154: 329-350; Zoller & Smith, Oligonucleotide-directed mutagenesis using M13-derived vectors: an efficient and general procedure for the production of point mutations in any DNA fragment, (1982) Nucleic Acids Res. 10:6487-6500; Zoller & Smith, Oligonucleotide-directed mutagenesis of DNA fragments cloned into M13 vectors, (1983) Methods in Enzymol. 100:468-500; and Zoller & Smith, Oligonucleotide-directed mutagenesis: a simple method using two oligonucleotide primers and a single-stranded DNA template, (1987) Methods in Enzymol. 154:329-350); phosphorothioate-modified DNA mutagenesis (Taylor et al., The use of phosphorothioate-modified DNA in restriction enzyme reactions to prepare nicked DNA, (1985) Nucl. Acids Res. 13: 8749-8764; Taylor et al., The rapid generation of oligonucleotide-directed mutations at high frequency using phosphorothioate-modified DNA, (1985) Nucl. Acids Res. 13: 8765-8787; Nakamaye & Eckstein, Inhibition of restriction endonuclease Nci I cleavage by phosphorothioate groups and its application to oligonucleotide-directed mutagenesis, (1986) Nucl. Acids Res. 14: 9679-9698; Sayers et al., Y-T Exonucleases in phosphorothioate-based oligonucleotide-directed mutagenesis, (1988) Nucl. Acids Res. 16:791-802; and Sayers et al., Strand specific cleavage of phosphorothioate-containing DNA by reaction with restriction endonucleases in the presence of ethidium bromide, (1988) Nucl. Acids Res. 16: 803-814); mutagenesis using gapped duplex DNA (Kramer et al., The gapped duplex DNA approach to oligonucleotide-directed mutation construction, (1984) Nucl. Acids Res. 12: 9441-9456; Kramer & Fritz Oligonucleotide-directed construction of mutations via gapped duplex DNA, (1987) Methods in Enzymol. 154:350-367; Kramer et al., Improved enzymatic in vitro reactions in the gapped duplex DNA approach to oligonucleotide-directed construction of mutations, (1988) Nucl. Acids Res. 16: 7207; and Fritz et al., Oligonucleotide-directed construction of mutations: a gapped duplex DNA procedure without enzymatic reactions in vitro, (1988) Nucl. Acids Res. 16: 6987-6999).

Additional suitable methods include point mismatch repair (Kramer et al., Point Mismatch Repair, (1984) Cell 38:879-887), mutagenesis using repair-deficient host strains (Carter et al., Improved oligonucleotide site-directed mutagenesis using M13 vectors, (1985) Nucl. Acids Res. 13: 4431-4443; and Carter, Improved oligonucleotide-directed mutagenesis using M13 vectors, (1987) Methods in Enzymol. 154: 382-403), deletion mutagenesis (Eghtedarzadeh & Henikoff, Use of oligonucleotides to generate large deletions, (1986) Nucl. Acids Res. 14: 5115), restriction-selection and restriction-purification (Wells et al., Importance of hydrogen-bond formation in stabilizing the transition state of subtilisin, (1986) Phil. Trans. R. Soc. Lond. A 317: 415-423), mutagenesis by total gene synthesis (Nambiar et al., Total synthesis and cloning of a gene coding for the ribonuclease S protein, (1984) Science 223: 1299-1301; Sakamar and Khorana, Total synthesis and expression of a gene for the a-subunit of bovine rod outer segment guanine nucleotide-binding protein (transducin), (1988) Nucl. Acids Res. 14: 6361-6372; Wells et al., Cassette mutagenesis: an efficient method for generation of multiple mutations at defined sites, (1985) Gene 34:315-323; and Grundström et al., Oligonucleotide-directed mutagenesis by microscale 'shot-gun' gene synthesis, (1985) Nucl. Acids Res. 13: 3305-3316), double-strand break repair (Mandecki, Oligonucleotide-directed double-strand break repair in plasmids of Escherichia coli: a method for site-specific mutagenesis, (1986) Proc. Natl. Acad. Sci. USA, 83:7177-7181; and Arnold, Protein engineering for unusual environments, (1993) Current Opinion in Biotechnology 4:450-455). Additional details on many of the above methods can be found in Methods in Enzymology Volume 154, which also describes useful controls for troubleshooting problems with various mutagenesis methods.

Additional details regarding various diversity generating methods can be found in the following U.S. patents, PCT publications and applications, and EPO publications: U.S. Pat. No. 5,605,793 to Stemmer (February 25, 1997), "Methods for In Vitro Recombination;" U.S. Pat. No. 5,811,238 to Stemmer et al. (September 22, 1998) "Methods for Generating Polynucleotides having Desired Characteristics by Iterative Selection and Recombination;" U.S. Pat. No. 5,830,721 to Stemmer et al. (November 3, 1998), "DNA Mutagenesis by Random Fragmentation and Reassembly;" U.S. Pat. No. 5,834,252 to Stemmer, et al. (November 10, 1998) "End-Complementary Polymerase Reaction;" U.S. Pat. No. 5,837,458 to Minshull, et al. (November 17, 1998), "Methods and Compositions for Cellular and Metabolic Engineering;" WO 95/22625, Stemmer and Crameri, "Mutagenesis by Random Fragmentation and Reassembly;" WO 96/33207 by Stemmer and Lipschutz "End Complementary Polymerase Chain Reaction;" WO 97/20078 by Stemmer and Crameri "Methods for Generating Polynucleotides having Desired Characteristics by Iterative Selection and Recombination;" WO 97/35966 by Minshull and Stemmer, "Methods and Compositions for Cellular and Metabolic Engineering;" WO 99/41402 by Punnonen et al. "Targeting of Genetic Vaccine Vectors;" WO 99/41383 by Punnonen et al. "Antigen Library Immunization;" WO 99/41369 by Punnonen et al. "Genetic Vaccine Vector Engineering;" WO 99/41368 by Punnonen et al. "Optimization of Immunomodulatory Properties of Genetic Vaccines;" EP 752008 by Stemmer and Crameri, "DNA Mutagenesis by Random Fragmentation and Reassembly;" EP 0932670 by Stemmer "Evolving Cellular DNA Uptake by Recursive Sequence Recombination;" WO 99/23107 by Stemmer et al., "Modification of Virus Tropism and Host Range by Viral Genome Shuffling;" WO 99/21979 by Apt et al., "Human Papillomavirus Vectors;" WO 98/31837 by del Cardayre et al. "Evolution of Whole Cells and Organisms by Recursive Sequence Recombination;" WO 98/27230 by Patten and Stemmer, "Methods and Compositions for Polypeptide Engineering;" WO 98/27230 by Stemmer et al., "Methods for Optimization of Gene Therapy by Recursive Sequence Shuffling and Selection," WO 00/00632, "Methods for Generating Highly Diverse Libraries," WO 00/09679, "Methods for Obtaining in Vitro Recombined Polynucleotide Sequence Banks and Resulting Sequences," WO 98/42832 by Arnold et al., "Recombination of Polynucleotide Sequences Using Random or Defined Primers," WO 99/29902 by Arnold et al., "Method for Creating Polynucleotide and Polypeptide Sequences," WO 98/41653 by Vind, "An in Vitro Method for Construction of a DNA Library," WO 98/41622 by Borchert et al., "Method for Constructing a Library Using DNA Shuffling," and WO 98/42727 by Pati and Zarling, "Sequence Alterations using Homologous Recombination;" WO 00/18906 by Patten et al., "Shuffling of Codon-Altered Genes;" WO 00/04190 by del Cardayre et al. "Evolution of Whole Cells and Organisms by Recursive Recombination;" WO 00/42561 by Crameri et al., "Oligonucleotide Mediated Nucleic Acid Recombination;" WO 00/42559 by Selifonov and Stemmer "Methods of Populating Data Structures for Use in Evolutionary Simulations;" WO 00/42560 by Selifonov et al., "Methods for Making Character Strings, Polynucleotides & Polypeptides Having Desired Characteristics;" WO 01/23401 by Welch et al., "Use of Codon-Varied Oligonucleotide Synthesis for Synthetic Shuffling;" and PCT/US01/06775 "Single-Stranded Nucleic Acid Template-Mediated Recombination and Nucleic Acid Fragment Isolation" by Affholter.

Another aspect described herein includes the cloning and expression of monomer domains, selected monomer domains, multimers and/or selected multimers coding nucleic acids. Thus, multimer domains can be synthesized as a single protein using expression systems well known in the art. In addition to the many texts noted above, general texts which describe molecular biological techniques useful herein, including the use of vectors, promoters and many other topics relevant to expressing nucleic acids such as monomer domains, selected monomer domains, multimers and/or selected multimers, include Berger and Kimmel, Guide to Molecular Cloning Techniques, Methods in Enzymology volume 152 Academic Press, Inc., San Diego, CA (Berger); Sambrook et al., Molecular Cloning - A Laboratory Manual (2nd Ed.), Vol. 1-3, Cold Spring Harbor Laboratory, Cold Spring Harbor, New York, 1989 ("Sambrook") and Current Protocols in Molecular Biology, F.M. Ausubel et al., eds., Current Protocols, a joint venture between Greene Publishing Associates, Inc. and John Wiley & Sons, Inc., (supplemented through 1999) ("Ausubel")). Examples of techniques sufficient to direct persons of skill through *in vitro* amplification methods, useful in identifying, isolating and cloning monomer domains and multimers coding nucleic acids, including the polymerase chain reaction (PCR) the ligase chain reaction (LCR), Q∃-replicase amplification and other RNA polymerase mediated techniques (*e.g.*, NASBA), are found in Berger, Sambrook, and Ausubel, as well as *Mullis et al*., (1987) U.S. Patent No. 4,683,202; PCR Protocols A Guide to Methods and Applications (Innis et al. eds) Academic Press Inc. San Diego, CA (1990) (Innis); Arnheim & Levinson (October 1, 1990) C&EN 36-47; The Journal Of NIH Research (1991) 3, 81-94; (Kwoh et al. (1989) Proc. Natl. Acad. Sci. USA 86, 1173; Guatelli et al. (1990) Proc. Natl. Acad. Sci. USA 87, 1874; Lomell et al. (1989) J. Clin. Chem 35, 1826; Landegren et al., (1988) Science 241, 1077-1080; Van Brunt (1990) Biotechnology 8, 291-294; Wu and Wallace, (1989) Gene 4, 560; Barringer et al. (1990) Gene 89, 117, and Sooknanan and Malek (1995) Biotechnology 13: 563-564. Improved methods of cloning *in vitro* amplified nucleic acids are described in Wallace et al., U.S. Pat. No. 5,426,039. Improved methods of amplifying large nucleic acids by PCR are summarized in Cheng et al. (1994) Nature 369: 684-685 and the references therein, in which PCR amplicons of up to 40kb are generated. One of skill will appreciate that essentially any RNA can be converted into a double stranded DNA suitable for restriction digestion, PCR expansion and sequencing using reverse transcriptase and a polymerase. *See,* Ausubel, Sambrook and Berger, *all supra*.

The vectors described herein can be introduced into host cells, and monomer domains, selected monomer domains, multimers and/or selected multimers of the invention can be produced by recombinant techniques. Host cells are genetically engineered (i.e., transduced, transformed or transfected) with the vectors described herein which can be, for example, a cloning vector or an expression vector. The vector can be, for example, in the form of a plasmid, a viral particle, a phage, etc. The engineered host cells can be cultured in conventional nutrient media modified as appropriate for activating promoters, selecting transformants, or amplifying the monomer domain, selected monomer domain, multimer and/or selected multimer gene(s) of interest. The culture conditions, such as temperature, pH and the like, are those previously used with the host cell selected for expression, and will be apparent to those skilled in the art and in the references cited herein, including, e.g., Freshney (1994) Culture of Animal Cells, a Manual of Basic Technique, third edition, Wiley- Liss, New York and the references cited therein.

As described herein, the polypeptides of the invention can also be produced in non-animal cells such as plants, yeast, fungi, bacteria and the like. Indeed, as noted throughout, phage display is an especially relevant technique for producing such polypeptides. In addition to Sambrook, Berger and Ausubel, details regarding cell culture can be found in Payne et al. (1992) Plant Cell and Tissue Culture in Liquid Systems John Wiley & Sons, Inc. New York, NY; Gamborg and Phillips (eds) (1995) Plant Cell, Tissue and Organ Culture; Fundamental Methods Springer Lab Manual, Springer-Verlag (Berlin Heidelberg New York) and Atlas and Parks (eds) The Handbook of Microbiological Media (1993) CRC Press, Boca Raton, FL.

Also described herein is alteration of monomer domains, and/or multimers to improve pharmacological properties, to reduce immunogenicity, or to facilitate the transport of the multimer and/or monomer domain into a cell or tissue (e.g., through the blood-brain barrier, or through the skin). These types of alterations include a variety of modifications (e.g., the addition of sugar-groups or glycosylation), the addition of PEG, the addition of protein domains that bind a certain protein (e.g., HAS or other serum protein), the addition of proteins fragments or sequences that signal movement or transport into, out of and through a cell. Additional components can also be added to a multimer and/or monomer domain to manipulate the properties of the multimer and/or monomer domain. A variety of components can also be added including, e.g., a domain that binds a known receptor (e.g., a Fc-region protein domain that binds a Fc receptor), a toxin(s) or part of a toxin, a prodomain that can be optionally cleaved off to activate the multimer or monomer domain, a reporter molecule (e.g., green fluorescent protein), a component that bind a reporter molecule (such as a radionuclide for radiotherapy, biotin or avidin) or a combination of modifications.

### 5. Kits - Described herein for Reference Only

Kits comprising the components needed in the methods (typically in an unmixed form) and kit components (packaging materials, instructions for using the components and/or the methods, one or more containers (reaction tubes, columns, etc.)) for holding the components are described herein. Kits may contain a multimer library, or a single type of multimer. Kits can also include reagents suitable for promoting target molecule binding, such as buffers or reagents that facilitate detection, including detectably-labeled molecules. Standards for calibrating a ligand binding to a monomer domain or the like, can also be included in the kits of the invention.

Also described herein are commercially valuable binding assays and kits to practice the assays. In some of the assays one or more ligand is employed to detect binding of a monomer domain, and/or multimer. Such assays are based on any known method in the art, e.g., flow cytometry, fluorescent microscopy, plasmon resonance, and the like, to detect binding of a ligand(s) to the monomer domain and/or multimer.

Kits based on the assay are also provided. The kits typically include a container, and one or more ligand. The kits optionally comprise directions for performing the assays, additional detection reagents, buffers, or instructions for the use of any of these components, or the like. Alternatively, kits can include cells, vectors, (e.g., expression vectors, secretion vectors comprising a polypeptide of the invention), for the expression of a monomer domain and/or a multimer of the invention.

### 6. Integrated Systems - Described for Reference Only

Also described herein are computers, computer readable media and integrated systems comprising character strings corresponding to monomer domains, selected monomer domains, multimers and/or selected multimers and nucleic acids encoding such polypeptides. These sequences can be manipulated by in silico shuffling methods, or by standard sequence alignment or word processing software.

For example, different types of similarity and considerations of various stringency and character string length can be detected and recognized in the integrated systems herein. For example, many homology determination methods have been designed for comparative analysis of sequences ofbiopolymers, for spell checking in word processing, and for data retrieval from various databases. With an understanding of double-helix pairwise complement interactions among 4 principal nucleobases in natural polynucleotides, models that simulate annealing of complementary homologous polynucleotide strings can also be used as a foundation of sequence alignment or other operations typically performed on the character strings corresponding to the sequences herein (e.g., word-processing manipulations, construction of figures comprising sequence or subsequence character strings, output tables, etc.). An example of a software package with GOs for calculating sequence similarity is BLAST, which can be adapted to the present invention by inputting character strings corresponding to the sequences herein.

BLAST is described in Altschul et al., (1990) J. Mol. Biol. 215:403-410. Software for performing BLAST analyses is publicly available through the National Center for Biotechnology Information (available on the World Wide Web at ncbi.nlm.nih.gov). This algorithm involves first identifying high scoring sequence pairs (HSPs) by identifying short words of length W in the query sequence, which either match or satisfy some positive-valued threshold score T when aligned with a word of the same length in a database sequence. T is referred to as the neighborhood word score threshold (Altschul *et al., supra*). These initial neighborhood word hits act as seeds for initiating searches to find longer HSPs containing them. The word hits are then extended in both directions along each sequence for as far as the cumulative alignment score can be increased. Cumulative scores are calculated using, for nucleotide sequences, the parameters M (reward score for a pair of matching residues; always > 0) and N (penalty score for mismatching residues; always < 0). For amino acid sequences, a scoring matrix is used to calculate the cumulative score. Extension of the word hits in each direction are halted when: the cumulative alignment score falls off by the quantity X from its maximum achieved value; the cumulative score goes to zero or below, due to the accumulation of one or more negative-scoring residue alignments; or the end of either sequence is reached. The BLAST algorithm parameters W, T, and X determine the sensitivity and speed of the alignment. The BLASTN program (for nucleotide sequences) uses as defaults a wordlength (W) of 11, an expectation (E) of 10, a cutoff of 100, M=5, N=-4, and a comparison of both strands. For amino acid sequences, the BLASTP program uses as defaults a wordlength (W) of 3, an expectation (E) of 10, and the BLOSUM62 scoring matrix (*see* Henikoff & Henikoff (1989) Proc. Natl. Acad. Sci. USA 89:10915).

An additional example of a useful sequence alignment algorithm is PILEUP. PILEUP creates a multiple sequence alignment from a group of related sequences using progressive, pairwise alignments. It can also plot a tree showing the clustering relationships used to create the alignment. PILEUP uses a simplification of the progressive alignment method of Feng & Doolittle, (1987) J. Mol. Evol. 35:351-360. The method used is similar to the method described by Higgins & Sharp, (1989) CABIOS 5:151-153. The program can align, e.g., up to 300 sequences of a maximum length of 5,000 letters. The multiple alignment procedure begins with the pairwise alignment of the two most similar sequences, producing a cluster of two aligned sequences. This cluster can then be aligned to the next most related sequence or cluster of aligned sequences. Two clusters of sequences can be aligned by a simple extension of the pairwise alignment of two individual sequences. The final alignment is achieved by a series of progressive, pairwise alignments. The program can also be used to plot a dendogram or tree representation of clustering relationships. The program is run by designating specific sequences and their amino acid or nucleotide coordinates for regions of sequence comparison. For example, in order to determine conserved amino acids in a monomer domain family or to compare the sequences of monomer domains in a family, the sequence of the invention, or coding nucleic acids, are aligned to provide structure-function information.

In one aspect described herein the computer system is used to perform "in silico" sequence recombination or shuffling of character strings corresponding to the monomer domains. A variety of such methods are set forth in "Methods For Making Character Strings, Polynucleotides & Polypeptides Having Desired Characteristics" by Selifonov and Stemmer, filed February 5, 1999 (USSN 60/118854) and "Methods For Making Character Strings, Polynucleotides & Polypeptides Having Desired Characteristics" by Selifonov and Stemmer, filed October 12, 1999 (USSN 09/416,375). In brief, genetic operators are used in genetic algorithms to change given sequences, e.g., by mimicking genetic events such as mutation, recombination, death and the like. Multi-dimensional analysis to optimize sequences can be also be performed in the computer system, e.g., as described in the '375 application.

A digital system can also instruct an oligonucleotide synthesizer to synthesize oligonucleotides, e.g., used for gene reconstruction or recombination, or to order oligonucleotides from commercial sources (e.g., by printing appropriate order forms or by linking to an order form on the Internet).

The digital system can also include output elements for controlling nucleic acid synthesis (*e.g*., based upon a sequence or an alignment of a recombinant, e.g., shuffled, monomer domain as herein), i.e., an integrated system of the invention optionally includes an oligonucleotide synthesizer or an oligonucleotide synthesis controller. The system can include other operations that occur downstream from an alignment or other operation performed using a character string corresponding to a sequence herein, e.g., as noted above with reference to assays.

### EXAMPLES

The following example is offered to illustrate, but not to limit the claimed invention.

### Example 1

This example describes selection of monomer domains and the creation of multimers.

Starting materials for identifying monomer domains and creating multimers from the selected monomer domains and procedures can be derived from any of a variety of human and/or non-human sequences. For example, to produce a selected monomer domain with specific binding for a desired ligand or mixture of ligands, one or more monomer domain gene(s) are selected from a family of monomer domains that bind to a certain ligand. The nucleic acid sequences encoding the one or more monomer domain gene can be obtained by PCR amplification of genomic DNA or cDNA, or optionally, can be produced synthetically using overlapping oligonucleotides.

Most commonly, these sequences are then cloned into a cell surface display format (i.e., bacterial, yeast, or mammalian (COS) cell surface display; phage display) for expression and screening. The recombinant sequences are transfected (transduced or transformed) into the appropriate host cell where they are expressed and displayed on the cell surface. For example, the cells can be stained with a labeled (e.g., fluorescently labeled), desired ligand. The stained cells are sorted by flow cytometry, and the selected monomer domains encoding genes are recovered (e.g., by plasmid isolation, PCR or expansion and cloning) from the positive cells. The process of staining and sorting can be repeated multiple times (e.g., using progressively decreasing concentrations of the desired ligand until a desired level of enrichment is obtained). Alternatively, any screening or detection method known in the art that can be used to identify cells that bind the desired ligand or mixture of ligands can be employed.

The selected monomer domain encoding genes recovered from the desired ligand or mixture of ligands binding cells can be optionally recombined according to any of the methods described herein or in the cited references. The recombinant sequences produced in this round of diversification are then screened by the same or a different method to identify recombinant genes with improved affinity for the desired or target ligand. The diversification and selection process is optionally repeated until a desired affinity is obtained.

The selected monomer domain nucleic acids selected by the methods can be joined together via a linker sequence to create multimers, e.g., by the combinatorial assembly of nucleic acid sequences encoding selected monomer domains by DNA ligation, or optionally, PCR-based, self priming overlap reactions. The nucleic acid sequences encoding the multimers are then cloned into a cell surface display format (i.e., bacterial, yeast, or mammalian (COS) cell surface display; phage display) for expression and screening. The recombinant sequences are transfected (transduced or transformed) into the appropriate host cell where they are expressed and displayed on the cell surface. For example, the cells can be stained with a labeled, e.g., fluorescently labeled, desired ligand or mixture of ligands. The stained cells are sorted by flow cytometry, and the selected multimers encoding genes are recovered (e.g., by PCR or expansion and cloning) from the positive cells. Positive cells include multimers with an improved avidity or affinity or altered specificity to the desired ligand or mixture of ligands compared to the selected monomer domain(s). The process of staining and sorting can be repeated multiple times (e.g., using progressively decreasing concentrations of the desired ligand or mixture of ligands until a desired level of enrichment is obtained). Alternatively, any screening or detection method known in the art that can be used to identify cells that bind the desired ligand or mixture of ligands can be employed.

The selected multimer encoding genes recovered from the desired ligand or mixture of ligands binding cells can be optionally recombined according to any of the methods described herein or in the cited references. The recombinant sequences produced in this round of diversification are then screened by the same or a different method to identify recombinant genes with improved avidity or affinity or altered specificity for the desired or target ligand. The diversification and selection process is optionally repeated until a desired avidity or affinity or altered specificity is obtained.

### Example 2

This example describes the development of a library of multimers comprised of C2 domains.

A library of DNA sequences encoding monomeric C2 domains is created by assembly PCR as described in Stemmer et al., Gene 164, 49-53 (1995). The oligonucleotides used in this PCR reaction are:

5'-acactgcaatcgcgccttacggctCCCGGGCGGATCCtcccataagttca
5'-agctaccaaagtgacannknnknnknnknnknnknnknnknnknnkccatacgtcgaattgttca
t
5'-agctaccaaagtgacaaaaggtgcttttggtgatatgttggatactccagatccatacgtcgaattgttca
t
5'-taggaagagaacacgtcattttnnknnknnkattaaccctgtttggaacgagacctttgagt
5'-taggaagagaacacgtcattttaataatgatattaaccctgtttggaacgagacctttgagt
5'-ttggaaatcaccctaatgnnknnknnknnknnknnknnkactctaggtacagcaa
5'-ttggaaatcaccctaatggatgcaaattatgttatggacgaaactctaggtacagcaa
5'-aagaaggaagtcccatttattttcaatcaagttactgaaatggtcttagagatgtccctt
5'-tgtcactttggtagctcttaacacaactacagtgaacttatgggaGGA
5'-acgtgttctcttcctagaatctggagttgtactgatgaacaattcgacgta
5'-attagggtgatttccaaaacattttcttgattaggatctaatataaactcaaaggtctcgtt
5'-atgggacttccttcttttctcccactttcattgaagatacagtaaacgttgctgtacctagagt
5'-gaccgatagcttgccgattgcagtgtGGCCACAGAGGCCTCGAGaacttcaaggacatctctaaga

PCR fragments are digested with BamHI and XhoI. Digestion products are separated on 1.5% agarose gel and C2 domain fragments are purified from the gel. The DNA fragments are ligated into the corresponding restriction sites of yeast surface display vector pYD1 (Invitrogen)

The ligation mixture is used for transformation of yeast strain EBY100. Transformants are selected by growing the cells in glucose-containing selective medium (-Trp) at 30°C.

Surface display of the C2 domain library is induced by growing the cells in galactose-containing selective medium at 20°C. Cells are rinsed with PBS and then incubated with fluorescently-labeled target protein and rinsed again in PBS.

Cells are then sorted by FACS and positive cells are regrown in glucose-containing selective medium. The cell culture may be used for a second round of sorting or may be used for isolation of plasmid DNA. Purified plasmid DNA is used as a template to PCR amplify C2 domain encoding DNA sequences.

The oligonucleotides used in this PCR reaction are:

5'-acactgcaatcgcgccttacggctCAGgtgCTGgtggttcccataagttcactgta
5'-gaccgatagcttgccgattgcagtCAGcacCTGaaccaccaccaccagaaccaccaccaccaacttcaa
gggacatctcta (linker sequence is underlined).

PCR fragments are then digested with AlwNI, digestion products are separated on 1.5% agarose gel and C2 domain fragments are purified from the gel. Subsequently, PCR fragments are multimerized by DNA ligation in the presence of stop fragments. The stop fragments are listed below:
Stop1: 
5'-gaattcaacgctactaccattagtagaattgatgccaccttttcagctcgcgccccaaat
gaaaaaatggtcaaactaaatctactcgttcgcagaattgggaatcaactgttacatggaatgaaacttccagac
accgtactttatgaatatttatgacgattccgaggcgcgcccggactacccgtatgatgttccggattatgcccc
gggatcctcaggtgctg-3' (digested with EcoRI and AlwNI).
Stop2:

5′-caggtgctgcactcgaggccactgcggccgcatattaacgtagattttcctccc
aacgtcctgactggtataatgagccagttcttaaaatcgcataaccagtacatggtgattaaagttgaaattaaa
ccgtctcaagagctttgttacgttgatttgggtaatgaagctt-3′ (digested with AlwNI and
HindIII).

The ligation mixture is then digested with EcoRI and HindIII.

Multimers are separated on 1% agarose gel and DNA fragments corresponding to stop1-C2-C2-stop2 are purified from the gel. Stop1-C2-C2-stop2 fragments are PCR amplified using primers 5' aattcaacgctactaccat-3' and 5'-agcttcattacccaaatcaac-3' and subsequently digested with BamHI and XhoI. Optionally, the polynucleotides encoding the multimers can be put through a further round of affinity screening (e.g., FACS analysis as described above).

Subsequently, high affinity binders are isolated and sequenced. DNA encoding the high binders is cloned into expression vector and replicated in a suitable host. Expressed proteins are purified and characterized.

### Example 3

This example describes the development of a library of trimers comprised of LDL receptor A domains.

A library of DNA sequences encoding monomeric A domains is created by assembly PCR as described in Stemmer et al., Gene 164, 49-53 (1995). The oligonucleotides used in this PCR reaction are: 
5'-CACTATGCATGGACTCAGTGTGTCCGATAAGGGCACACGGTGCCTACCCGTATGATGTTCCGGATTATGCC
CCGGGCAGTA
5'-CGCCGTCGCATMSCMAGYKCNSAGRAATACAWYGGCCGYTWYYGCACBKAAATTSGYYAGVCNSACAGGTA
CTGCCCGGGGCAT
5'-CGCCGTCGCATMSCMATKCCNSAGRAATACAWYGGCCGYTWYYGCACBKAAATTSGYYAGVCNSACAGGTA
CTGCCCGGGGCAT
5'-ATGCGACGGCGWWRATGATTGTSVAGATGGTAGCGATGAAVWGRRTTGTVMAVNMVNMVGCCVTACGGGCT
CGGCCTCT
5'-ATGCGACGGCGWWCCGGATTGTSVAGATGGTAGCGATGAAVWGRRTTGTVMAVNMVNMVGCCVTACGGGCT
CGGCCTCT
5'-ATGCGACGGCGWWRATGATTGTSVAGATAACAGCGATGAAVWGRRTTGTVMAVNMVNMVGCCVTACGGGCT
CGGCCTCT
5'-ATGCGACGGCGWWCCGGATTGTSVAGATAACAGCGATGAAVWGRRTTGTVMAVNMVNMVGCCVTACGGGCT
CGGCCTCT
5'-TCCTGGTAGTACTTATCTACTACTATTTGTCTGTGTCTGCTCTGGGTTCCTAACGGTTCGGCCACAGAGGC
CGAGCCCGTA
 where R=A/G, Y=C/T, M=A/C, K=G/T, S=C/G, W=A/T, B=C/G/T, D=A/G/T, H=A/C/T, V=A/C/G, and N=A/C/G/T.

PCR fragments are digested with XmaI and SfiI. Digestion products are separated on 3% agarose gel and A domain fragments are purified from the gel. The DNA fragments are then ligated into the corresponding restriction sites of phage display vector fuse5-HA, a derivative of fuse5. The ligation mixture is electroporated into electrocompetent E. *coli* cells (F- strain e.g. Top10 or MC1061). Transformed *E. coli* cells are grown overnight in 2xYT medium containing 20 µg/ml tetracycline.

Virions are purified from this culture by PEG-precipitation. Target protein is immobilized on solid surface (e.g. petridish or microtiter plate) directly by incubating in 0.1 M NaHCO₃ or indirectly via a biotin-streptavidin linkage. Purified virions are added at a typical number of ∼1-3 x 10¹¹ TU. The petridish or microtiter plate is incubated at 4°C, washed several times with washing buffer (TBS/Tween) and bound phages are eluted by adding glycine.HCl buffer. The eluate is neutralized by adding 1 M Tris-HCl (pH 9.1)

The phages are amplified and subsequently used as input to a second round of affinity selection. ssDNA is extracted from the final eluate using QIAprep M13 kit. ssDNA is used as a template to PCR amplify A domains encoding DNA sequences.

The oligonucleotides used in this PCR reaction are:
5'-aagcctcagcgaccgaa
5'-agcccaataggaacccat

PCR fragments are digested with AlwNI and BgII. Digestion products are separated on 3% agarose gel and A domain fragments are purified from the gel. PCR fragments are multimerized by DNA ligation in the presence of the following stop fragments:
Stop 1: 
5'-gaattcaacgctactaccattagtagaattgatgccacctttttcagctcgcgccccaaatgaaaaaatggt
caaactaaatctactcgttcgcagaattgggaatcaactgttacatggaatgaaacttccagacaccgtacttta
tgaatatttatgacgattccgaggcgcgcccggactacccgtatgatgttccggattatgccccgggcggatcca
gtacctg-3' (digested with EcoRI and ALwNI)
Stop2:

5'-gccctacgggcctcgaggcacctggtgcggccgcatattaacgtagattttcctcccaacgtcctgactg
gtataatgagccagttcttaaaatcgcataaccagtacatggtgattaaagttgaaattaaaccgtctcaagagc
tttgttacgttgatttgggtaatgaagctt-3' (digested with BglI and HindIII)

The ligation mixture is digested with EcoRI and HindIII.

Multimers are separated on 1% agarose gel and DNA fragments corresponding to stop1-A-A-A-stop2 are purified from the gel. Stop1-A-A-A-stop2 fragments are subsequently PCR amplified using primers 5'-agcttcattacccaaatcaac-3' and 5' aattcaacgctactaccat-3' and subsequently digested with XmaI and SfiI. Selected polynucleotides are then cloned into a phage expression system and tested for affinity for the target protein.

High affinity binders are subsequently isolated and sequenced. DNA encoding the high binders is cloned into expression vector and subsequently expressed in a suitable host. The expressed protein is then purified and characterized.

## Claims

1. A method for identifying a multimer that binds to a target molecule, the method comprising,
(a) providing a library of polypeptides, the polypeptides comprising different monomer domains, wherein each monomer domain has 30-100 amino acids, comprises at least two disulfide bonds, and is an LDL-receptor class A domain;
(b) screening the library of polypeptides for affinity to a target molecule;
(c) identifying at least a first polypeptide comprising a first monomer domain that specifically binds to a target molecule;
(d) linking the first monomer domain to a plurality of different monomer domains to form a library of different multimers, the multimers comprising the first monomer domain and one of the plurality of different monomer domains;
(e) screening the library of multimers for the ability to bind to the target molecule; and
(f) identifying a multimer that specifically binds to the target molecule, wherein the multimer comprises the first monomer domain and a second monomer domain.

2. The method of claim 1, wherein the selected multimer comprises at least three monomer domains.

3. The method of any preceding claim, wherein the library of multimers is expressed as a phage display, ribosome display or cell surface display.

4. The method of any preceding claim, wherein the monomer domains are linked by a heterologous polypeptide linker.

5. A method for identifying a multimer that binds to at least one target molecule, the method comprising:
(a) providing a library of multimers, wherein each multimer comprises at least two monomer domains, wherein each monomer domain has 30-100 amino acids and comprises at least two disulfide bonds, separated by a heterologous linker wherein the monomer domains are LDL-receptor class A domains; and
(b) screening the library of multimers for target molecule-binding multimers.

6. The method of claim 5, further comprising identifying target molecule-binding multimers having an avidity for the target molecule that is greater than the avidity of a single monomer domain for the target molecule.

7. The method of claim 5 or claim 6, wherein one or more of the multimers comprises a monomer domain that specifically binds to a second target molecule.

8. A library of multimers, wherein
(a) each multimer comprises at least two monomer domains connected by a heterologous linker, wherein each monomer domain has 30-100 amino acids and comprises at least two disulfide bonds; and
(b) the monomer domains are LDL-receptor class A domains.

9. The library of claim 8, wherein each monomer domain of the multimers is a non-naturally occurring monomer domain.

10. The library of claim 8, wherein the monomer domains are linked by a polypeptide linker.

11. The method or library of any preceding claim, wherein the library contains at least 100 members.

12. A polypeptide comprising at least two monomer domains separated by a heterologous linker, wherein each monomer domain has 30-100 amino acids and comprises at least two disulfide bonds, wherein each monomer domain specifically binds to a target molecule and wherein the monomer domains are LDL-receptor class A domains.

13. The polypeptide of claim 12, wherein each monomer domain is a non-naturally occurring protein monomer domain.

14. The polypeptide of claim 12, wherein the polypeptide comprises a first monomer domain that binds a first target molecule and a second monomer domain that binds a second target molecule.

15. The polypeptide of claim 12, wherein the polypeptide comprises two monomer domains, each monomer domain having a binding specificity for a different site on a first target molecule.

16. The polypeptide of claim 12, wherein the polypeptide comprises at least three monomer domains.

17. The polypeptide of claim 12, wherein the monomer domains are linked by a polypeptide linker.

## Patentansprüche

1. Verfahren zur Identifizierung eines Multimers, das an ein Zielmolekül bindet, wobei das Verfahren folgende Schritte aufweist, nämlich
(a) Bereitstellen einer Bibliothek von Polypeptiden, wobei die Polypeptide verschiedene Monomerdomänen aufweisen, wobei jede Monomerdomäne 30-100 Aminosäuren aufweist, zumindest zwei Disulfidbrücken aufweist und eine Domäne eines LDL-Rezeptors der Klasse A ist;
(b) Screenen der Bibliothek von Polypeptiden auf eine Affinität zu einem Zielmolekül;
(c) Identifizieren von zumindest einem ersten Polypeptid, das eine erste Monomerdomäne aufweist, die spezifisch an ein Zielmolekül bindet;
(d) Verbinden der ersten Monomerdomäne mit einer Vielzahl von verschiedenen Monomerdomänen, um eine Bibliothek von verschiedenen Multimeren zu bilden, wobei die Multimere die erste Monomerdomäne aufweisen und eine der Vielzahl von verschiedenen Monomerdomänen;
(e) Screenen der Bibliothek von Multimeren nach deren Fähigkeit, an das Zielmolekül zu binden, und
(f) Identifizieren eines Multimers, das spezifisch an das Zielmolekül bindet, wobei das Multimer die erste Monomerdomäne und eine zweite Monomerdomäne aufweist.

2. Verfahren nach Anspruch 1, wobei das ausgewählte Multimer zumindest drei Monomerdomänen aufweist.

3. Verfahren nach einem der vorherigen Ansprüche, wobei die Bibliothek von Multimeren als Phagendisplay, Ribosomendisplay oder Zelloberflächendisplay exprimiert wird.

4. Verfahren nach einem der vorherigen Ansprüche, wobei die Monomerdomänen über einen heterologen Polypeptidlinker verbunden sind.

5. Verfahren zur Identifizierung eines Multimers, das an zumindest ein Zielmolekül bindet, wobei das Verfahren folgende Schritte aufweist, nämlich
(a) Bereitstellen einer Bibliothek von Multimeren, wobei jedes Multimer zumindest zwei Monomerdomänen aufweist, wobei jede Monomerdomäne 30 bis 100 Aminosäuren aufweist und zumindest zwei Disulfidbrücken aufweist, die durch einen heterologen Linker getrennt sind, wobei die Monomerdomänen solche Domänen eines LDL-Rezeptors der Klasse A sind, und
(b) Screenen der Bibliothek von Multimeren auf an das Zielmolekül bindende Multimere.

6. Verfahren nach Anspruch 5, das ferner das Identifizieren von an das Zielmolekül bindenden Multimeren aufweist, die eine Avidität zu dem Zielmolekül aufweisen, die größer ist als die Avidität einer einzelnen Monomerdomäne zu dem Zielmolekül.

7. Verfahren nach Anspruch 5 oder 6, wobei ein oder mehrere der Multimere eine Monomerdomäne aufweisen, die spezifisch an ein zweites Zielmolekül bindet.

8. Bibliothek von Multimeren, wobei
(a) jedes Multimer zumindest zwei Monomerdomänen aufweist, die über einen heterologen Linker verbunden sind, wobei jede Monomerdomäne 30-100 Aminosäuren aufweist und zumindest zwei Disulfidbrücken aufweist, und
(b) die Monomerdomänen solche Domänen eines LDL-Rezeptors der Klasse A sind.

9. Bibliothek nach Anspruch 8, wobei jede Monomerdomäne des Multimers eine nicht natürlich vorkommende Monomerdomäne ist.

10. Bibliothek nach Anspruch 8, wobei die Monomerdomänen über einen Polypeptidlinker verbunden sind.

11. Verfahren oder Bibliothek nach einem der vorherigen Ansprüche, wobei die Bibliothek zumindest 100 Mitglieder enthält.

12. Polypeptid, das zumindest zwei Monomerdomänen aufweist, die durch einen heterologen Linker getrennt sind, wobei jede Monomerdomäne 30-100 Aminosäuren aufweist und zumindest zwei Disulfidbrücken aufweist, wobei jede Monomerdomäne spezifisch an ein Zielmolekül bindet, und wobei die Monomerdomänen solche Domänen eines LDL-Rezeptors der Klasse A sind.

13. Polypeptid nach Anspruch 12, wobei jede Monomerdomäne eine nicht natürlich vorkommende Proteinmonomerdomäne ist.

14. Polypeptid nach Anspruch 12, wobei das Polypeptid eine erste Monomerdomäne, die an ein erstes Zielmolekül bindet, und eine zweite Domäne, die an ein zweites Zielmolekül bindet, aufweist.

15. Polypeptid nach Anspruch 12, wobei das Polypeptid zwei Monomerdomänen aufweist, wobei jede Monomerdomäne eine Bindungsspezifität für eine unterschiedliche Stelle in einem ersten Zielmolekül aufweist.

16. Polypeptid nach Anspruch 12, wobei das Polypeptid zumindest drei Monomerdomänen aufweist.

17. Polypeptid nach Anspruch 12, wobei die Monomerdomänen über einen Polypeptidlinker verbunden sind.

## Revendications

1. Procédé pour identifier un multimère qui se lie à une molécule cible, le procédé comprenant :
(a) mettre à disposition une banque de polypeptides, les polypeptides comprenant différents domaines monomériques, où chaque domaine monomérique possède 30-100 acides aminés, comprend au moins deux liaisons disulfures, et est un domaine du récepteur des LDL de classe A ;
(b) cribler la banque de polypeptides pour identifier une affinité avec une molécule cible ;
(c) identifier au moins un premier polypeptide comprenant un premier domaine monomérique qui se lie spécifiquement à une molécule cible ;
(d) lier le premier domaine monomérique à une pluralité de domaines monomériques différents pour former une banque de différents multimères, les multimères comprenant le premier domaine monomérique et l'un des différents domaines monomériques différents ;
(e) cribler la banque de multimères pour leur aptitude à se lier à la molécule cible ; et
(f) identifier un multimère qui se lie spécifiquement à la molécule cible, où le multimère comprend le premier domaine monomérique et un second domaine monomérique.

2. Procédé selon la revendication 1, dans lequel le multimère sélectionné comprend au moins trois domaines monomériques.

3. Procédé selon l'une quelconque des revendications précédentes, dans lequel la banque de multimères est exprimée sous forme de présentation sur phages (phage display), présentation par un ribosome ou présentation à la surface cellulaire.

4. Procédé selon l'une quelconque des revendications précédentes, dans lequel les domaines monomériques sont liés par un polypeptide de liaison hétérologue.

5. Procédé pour identifier un multimère qui se lie à au moins une molécule cible, le procédé comprenant :
(a) mettre à disposition une banque de multimères, où chaque multimère comprend au moins deux domaines monomériques, où chaque domaine monomérique possède 30-100 acides aminés et comprend au moins deux liaisons disulfures, séparées par un segment de liaison hétérologue où les domaines monomériques sont des domaines du récepteur des LDL de classe A ; et
(b) cribler la banque de multimères pour identifier des multimères se liant à une molécule cible.

6. Procédé selon la revendication 5, comprenant en outre identifier des multimères se liant à une molécule cible ayant une avidité pour la molécule cible qui est supérieure à l'avidité d'un seul domaine monomérique pour la molécule cible.

7. Procédé selon la revendication 5 ou la revendication 6, dans lequel un ou plusieurs des multimères comprend un domaine monomérique qui se lie spécifiquement à une seconde molécule cible.

8. Banques de multimères, dans laquelle
(a) chaque multimère comprend au moins deux domaines monomériques reliés par un segment de liaison hétérologue, où chaque domaine monomérique possède 30-100 acides aminés et comprend au moins deux liaisons disulfures ; et
(b) les domaines monomériques sont des domaines du récepteur des LDL de classe A.

9. Banque selon la revendication 8, dans laquelle chaque domaine monomérique des multimères est un domaine monomérique qui n'existe pas à l'état naturel.

10. Banque selon la revendication 8, dans laquelle les domaines monomériques sont liés par un polypeptide de liaison.

11. Procédé ou banque selon l'une quelconque des revendications précédentes, où la banque contient au moins 100 membres.

12. Polypeptide comprenant au moins deux domaines monomériques séparés par un segment de liaison hétérologue, où chaque domaine monomérique possède 30-100 acides aminés et comprend au moins deux liaisons disulfures, où chaque domaine monomérique se lie spécifiquement à une molécule cible et où les domaines monomériques sont des domaines du récepteur des LDL de classe A.

13. Polypeptide selon la revendication 12, dans lequel chaque domaine monomérique est un domaine monomérique protéique qui n'existe pas à l'état naturel.

14. Polypeptide selon la revendication 12, où le polypeptide comprend un premier domaine monomérique qui se lie à une première molécule cible et un second domaine monomérique qui se lie à une seconde molécule cible.

15. Polypeptide selon la revendication 12, où le polypeptide comprend deux domaines monomériques, chaque domaine monomérique ayant une spécificité de liaison pour un site différent sur une première molécule cible.

16. Polypeptide selon la revendication 12, où le polypeptide comprend au moins trois domaines monomériques.

17. Polypeptide selon la revendication 12, dans lequel les domaines monomériques sont liés par un polypeptide de liaison.
